# EUROPEAN PATENT SPECIFICATION

(11) **EP 1 904 073 B1**
(45) Date of publication and mention of the grant of the patent: **16.05.2018**
(21) Application number: 06809040.6
(22) Date of filing: 12.07.2006
(51) Int. Cl.: A61K 35/30

(54) **INFLAMMATION**
ENTZÜNDUNG
INFLAMMATION

(30) Priority: 12.07.2005 GB 0514300
(43) Date of publication of application: 02.04.2008
(73) Proprietor: Ospedale San Raffaele S.r.l., 20132 Milano (IT)
(72) Inventor: PLUCHINO, Stefano, Via Olgettina, 60, I-20132 Milan (IT); MARTINO, Gianvito, Via Olgettina, 60, I-20132 Milan (IT)
(74) Representative: D Young & Co LLP
(86) International application number: PCT/IB2006/002896
(87) International publication number: WO 2007/015173

(56) References cited:
- WO-A-01/79495
- WO-A1-99/49014
- WO-A2-03/070171
- PLUCHINO S ET AL: "Neural stem cells and their use as therapeutic tool in neurological disorders" BRAIN RESEARCH REVIEWS, ELSEVIER, XX, vol. 48, no. 2, April 2005 (2005-04), pages 211-219, XP004873187 ISSN: 0165-0173
- GUO ALEXANDER C ET AL: "Multiple sclerosis: Diffusion tensor MR imaging for evaluation of normal-appearing white matter" RADIOLOGY, vol. 222, no. 3, March 2002 (2002-03), pages 729-736, XP002426716 ISSN: 0033-8419
- PLUCHINO STEFANO ET AL: "Neurosphere-derived multipotent precursors promote neuroprotection by an immunomodulatory mechanism" NATURE (LONDON), vol. 436, no. 7048, July 2005 (2005-07), pages 266-271, XP002426967 ISSN: 0028-0836

## Description

### Field of the Invention

The present disclosure relates to the use of multipotent somatic stem cells for reducing inflammation, and in particular, to the use of adult neural stem cells (aNSC) for treating inflammation associated with central nervous system disorders and for inducing central and/or peripheral tolerance in a neurodegenerative disorder.

### Background of the Invention

A stem cell is an unspecialised cell which has the ability to renew itself indefinitely, and, under appropriate conditions, can give rise to a wide range of mature cell types in the human body. As any disorder involving loss of, or injury to, normal cells could be a candidate for stem cell replacement therapy, the potential of stem cells is profound. Organ and tissue generation from stem cells, and their subsequent transplantation provide promising treatments for a number of pathologies, including diabetes, central nervous system (CNS) disorders, liver disease, heart disease, and autoimmune disorders.

Stem cells have been identified in most organs or tissues. Perhaps the best characterised is the hematopoietic stem cell (HSC). The HSC, isolated from bone marrow, blood, cord blood, fetal liver and yolk sac, is the progenitor cell that generates blood cells or, following translation, reinitiates multiple hematopoietic lineages and can reinitiate hematopoiesis for the life of a recipient. (U.S. Patent No. 5,635,387 and U.S. Patent No. 5,460,964).

Another adult stem cell that has been studied is the aNSC. aNSCs were initially identified in the subventricular zone and the olfactory bulb of fetal brain. Until recently, it was believed that the adult brain no longer contained cells with stem cell potential. However, several studies in rodents, and more recently also non-human primates and humans, have shown that stem cells continue to be present in the adult brain. These stem cells can proliferate *in vivo* and continuously regenerate at least some neuronal cells *in vivo.*

Stem cell therapy for CNS disorders has generated particular interest because of their debilitating nature and widespread occurrence. CNS disorders encompass numerous acute and chronic afflictions which are characterized either by primary inflammation that leads to secondary neurodegeneration (for example, Multiple Sclerosis (MS), spinal cord injury (SCI), brain trauma and stroke), or by the primary neurodegeneration that is accompanied by secondary reactive inflammation (for example, Parkinson's Disease (PD), Alzheimer's Disease (AD), Huntington's Disease (HD) and epilepsy). The final net effect of such disorders is the loss and/or degeneration of neural cells in particular locations of the CNS, leading to the inability of these cells or the brain region to carry out their intended function which, in turn, causes behaviour abnormalities.

To date, the treatment of CNS disorders has been primarily via the administration of pharmaceutical compounds. Unfortunately, this type of treatment has been fraught with many complications including the limited ability to transport drugs across the blood-brain barrier and the drug-tolerance which is acquired by patients to whom these drugs are administered long-term. Thus, the use of stem cell has exciting potential in the treatment of these disorders. However, the transplantation of aNSCs in patients affected by CNS disorders characterised by chronic inflammation may have little therapeutic impact due to recurrent or persisting inflammation that may target and destroy both CNS-resident, as well as transplanted therapeutic, cells.

Inflammation is a self-defensive reaction aimed at eliminating or neutralising injurious stimuli, and restoring tissue integrity. Like peripheral inflammation, neuroinflammation can become a harmful process, and it is now widely accepted that it may contributes to the pathogenesis of many central nervous system disorders. CNS inflammation is commonly associated with some degree of tissue damage including, loss of myelin sheaths or loss of axons, and is a central theme in human patients with MS.

MS and its animal model, experimental autoimmune encephalomyelitis (EAE), are associated with inflammatory, delayed-type hypersensitivity responses, with cytokines playing a prominent role as mediators of intercellular signalling and effector function. Cell adhesion molecules, costimulator ligands and matrix metalloproteinases are also upregulated during the autoimmune response, and are implicated in leukocyte infiltration to the CNS (Owens et al., Neurol. Clin. 13, 51-73 (1995)). The inflammatory responses that arise as a result of tissue injury can contribute to further injury.

A number of cytokines, chemokines and related mediators have been implicated in EAE. Notably, the Th1-associated cytokines IFN-γ, TNF-α, IL-6 and the Th-1-inducing, cytokine IL-12 are associated with active disease or relapse, whereas the Th-2 cytokines IL-4, IL-10 and IL-13 are associated either with remission or suppression of the disease (Owens et al., Nature Medicine 7 161-166 (2001)).

There remains a need to develop therapies that are effective in reducing tissue inflammation. The present application addresses this problem.

### Summary of the Invention

The present invention surprisingly shows that stem cells have significant therapeutic application in chronic inflammatory CNS (and non-CNS) disorders by virtue of their ability to induce tissue protection by reducing inflammation.

We have shown that, upon systemic injection, undifferentiated adult stem cells promote brain repair by exerting previously unidentified immune-like functions. In particular, we have demonstrated that, in experimental inflammatory CNS disorders, such as relapsing-EAE (R-EAE) in the SJL mouse, transplantation of aNSCs exerts significant neuroprotection by inducing *in situ* (namely, in inflamed CNS perivascular areas) programmed cell death of pro-inflammatory Th1, but not anti-inflammatory Th2, blood-borne CNS-infiltrating cells,. Moreover, transplanted aNSCs survive to recurrent inflammatory episodes by retaining both an undifferentiated phenotype and notable proliferating and immunomodulatory capacities thus being able to protecting for long term from chronic neural tissue loss as well as disease-related disability [Pluchino et al., Nature 436: 266-271 (2005)]. Thus, the CNS inflammatory microenvironment dictates the fate (and the mechanism(s) of therapeutic efficacy) of aNSCs injected trough biological routes (e.g., cerebrospinal fluid, blood-stream). In certain circumstances, expecially when CNS degeneration prevails - as it is the case of chronic EAE (C-EAE) in B6 mice - transplanted aNSCs may also favour endogenous myelin-producing cells to acquire a mature phenotype and replace damaged neural cells [Pluchino et al., Nature 422: 688-694 (2003)].

Some of the advantages of using immunomodulatory adult stem cells for the treatment of CNS disorders are outlined below:
- Therapeutic adult stem cells can be grown in the presence of growth factors as undifferentiated cells, allowing the generation of virtually-indefinite numbers of transplantable cells.
- Intravenously-injected neural stem cells accumulate selectively within CNS inflamed perivascular areas using constitutively functional homing molecules (e.g., α4 integrins, CD44 and G-protein coupled receptors (GPCRs)), which are canonically used by pathogenic CNS-infiltrating blood borne lympho/monocytes.
- Within the CNS, transplanted neural stem cells accumulate within perivascular areas where reactive astrocytes, inflamed endothelial cells, and encephalitogenic T cells contribute to the establishment of CNS "atypical perivascular niches" through the focal release of major stem cell regulators. Within these de novo formed niche-like perivascular areas, transplanted aNSCs maintain preferentially an undifferentiated phenotype upon transplantation, are potentially able to escape from, and survive to repeated episodes of CNS inflammation.
- Considerable numbers (e.g. 7-8%) of transplanted neural stem cells also maintain capacity of proliferation in vivo, thus being potentially able to modulate their in vivo fate (proliferation vs quiescence vs migration and differentiation) in response to specific environmental signals.

The present invention relates to the stem cell-mediated immunomodulatory mechanisms. We have observed that adult neural stem cells display ability to induce both central as well as peripheral tolerance in inflammatory CNS diseases and neurodegenerative diseases. Indeed, we have shown that neural-committed stem cells are able to prevent or decrease inflammation in chronic inflammatory CNS diseases through either the induction of programmed cell death (apoptosis) of blood-borne CNS-infiltrating pro-inflammatory Th1 cells (*central tolerance*) (Pluchino et al (1995) Nature 436:266-271) or/and through a second immunomodulatory mechanism leading to immune tolerance in periphery (e.g. secondary lymphoid organs) tolerance in, for example, MS.

Thus, we envisage new methods of treating CNS and non-CNS inflammatory conditions with adult neural stem cells. Indeed adult neural stem cells rather than being therapeutically efficacious via massive cell replacement can be used as potent immunomodulators for preventing and/or inhibiting chronic inflammation in CNS (or other) inflammatory diseases.

According to one aspect of the present invention there is provided an adult neural stem cell (aNSC) for use in the treatment of inflammation associated with central nervous system disorders.

According to another aspect of the present invention there is provided use of an adult neural stem cell (aNSC) for the preparation of a medicament for the treatment of inflammation associated with central nervous system disorders.

According to one aspect of the present disclosure there is provided use of an aNSC for the preparation of a medicament for treating central nervous system disorders.

According to another aspect of the present disclosure there is provided a method of treating central nervous system disorders in a patient suffering from said disease which comprises administering to the patient a therapeutically effective amount of aNSCs.

According to another aspect of the present disclosure there is provided use of a stem cell for the preparation of a medicament for inducing central and/or peripheral tolerance.

According to another aspect of the present disclosure there is provided a method of inducing central and/or peripheral tolerance in a patient which comprises administering to the patient a therapeutic amount of a stem cell.

According to another aspect of the present disclosure there is provided use of a stem cell for the preparation of a medicament for inducing tissue protection by reducing inflammation.

According to another aspect of the present disclosure there is provided a method of reducing inflammation in a patient suffering from inflammation which comprises administering to the patient a therapeutically effective amount of stem cells.

Preferably the inflammation is associated with a chronic central nervous system disorder.

In one embodiment the disease is a systemic or organ- specific disorder characteristed by chronic inflammation, such a rheumatoid arthritis or type 1 diabetes.

According to another aspect of the present disclosure there is provided use of a stem cell for the preparation of a medicament for inducing apoptosis of central nervous system infiltrating pro-inflammatory T cells.

According to another aspect of the present disclosure there is provided a method of treating central nervous system disorders in a patient suffering from said disease which comprises inducing apoptosis of central nervous system infiltrating pro-inflammatory T cells by administering to the patient a therapeutically effective amount stem cells.

Preferably the pro-inflammatory T cells are blood-borne CD45⁺ inflammatory cells.

Preferably the stem cell of the disclosure is not an embryonic stem cell, more preferably not a human embryonic stem cell.

Preferably the stem cell of the disclosure is not a pluripotent cell, more preferably not a human pluripotent cell.

Preferably the stem cell of the disclosure is not a totipotent cell, more preferably not a human totipotent cell.

Preferably the stem cell of the disclosure is a multipotent stem cell.

In one embodiment of the disclosure the stem cell is a multipotent somatic stem cell.

Preferably the multipotent somatic stem cell of the disclosure is a neural cell.

Preferably the aNSC is derived from adult brain or spinal cord. This includes aNSC derived from foetal brain or spinal cord.

Preferably the aNSC is derived from the subventricular zone.

In one embodiment the stem cell as previously defined expresses a targeting moiety for a site of inflammation, such as a CNS inflamed lesion. The stem cell may be genetically modified to express the targeting moiety.

Preferably the stem cell expresses an integrin, a cell adhesion molecule (CAM) or a functional chemokine receptor that allow for the selective targeting of an inflamed area.

Preferably the integrin is α4 integrin very late antigen (VLA)-4.

Preferably the CAM is the CD44, a widely expressed molecule that binds hyaluronate.

Preferably the chemokine receptor is selected form the group comprising CCR2, CCR5, CXCR3 and CXCR4.

In another embodiment, the stem cell used in the present invention expresses a pro-apoptotic molecule. The stem cell may be genetically modified to express the pro-apoptotic molecule.

Preferably, the pro-apoptotic molecule is a major death receptor ligand, such as FasL, Apo3L and TRAIL.

In one embodiment the stem cells are mammalian - e.g. murine, human, primate, porcine, feline or canine.

In another embodiment, the medicament and/or treatment is given after onset of a central nervous system disorder.

Preferably the central nervous system disorder is a chronic central nervous system disorder.

Preferably the central nervous system disorder is a neurodegenerative disorder.

Examples of central nervous system disorders include, but are not limited to, dementia, multiple sclerosis, amyotrophic lateral sclerosis, Alzheimer's Disease, Huntington's Disease, Parkinson's Disease, brain tumour, acute spinal cord injury and ischemic stroke.

Preferably the stem cells used in the present invention are administered intravenously or intrathecally.

According to another aspect of the present disclosure there is provided a method of inducing tissue protection by reducing inflammation associated with central nervous system disorders (neuroinflammation) comprising:
(i) identifying the inflammation-related time window for stem cell administration in a patient suffering from a central nervous system disorder; and
(i) administrating stem cells to the patient.

Preferably the inflammation is identified using magnetic resonance imaging.

The stem cell used in the method of the disclosure may be any stem cell as defined above.

Preferably the stem cell used in the method of the disclosure is not an embryonic stem cell, more preferably not a human embryonic stem cell.

Preferably the stem cell used in the method of the disclosure is not a pluripotent cell, more preferably not a human pluripotent stem cell.

Preferably the stem cell used in the method of the disclosure is not a totipotent cell, more preferably not a human totipotent stem cell.

Preferably the stem cells are administered after the onset of a central nervous system disorder, preferably a chronic central nervous system disorder.

In one embodiment the central nervous system disorder treated by the method of the disclosure is selected from the group comprising multiple sclerosis, amyotrophic lateral sclerosis, Alzheimer's Disease, Huntington's Disease, Parkinson's Disease, brain tumour, acture spinal cord injury and ischemic stroke.

More preferably the central nervous system disorder is selected from the group comprising multiple sclerosis, brain tumour, spinal cord injury and ischemic stroke.

Preferably the stem cells administered in the method of the disclosure are administered intravenously or intrathecally.

### Detailed description

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; and E. M. Shevach and W. Strober, 1992 and periodic supplements, Current Protocols in Immunology, John Wiley & Sons, New York, NY.

### Stem cells

Stem cells are undifferentiated, primitive cells with the ability both to multiply for indefinite periods and differentiate into specific kinds of cells. Mammalian stem cells can be pluripotent cell lines derived from mammalian embryos, such as ES, EG or EC cells, or can be multipotent and derived from adults. Mammalian stem cells may be derived from any mammalian species, such as murine, human or other primate (e.g. chimpanzee, cynomolgus monkey, baboon, and other Old World monkey), porcine, canine, equine and feline.

Pluripotent stem cells are stem cells, with the potential to make any differentiated cell in the body.

Multipotent stem cells are characterised as undifferentiated cells with the capacity for extensive proliferation that gives rise to more stem cells (exhibit self-renewal) as well as progeny that will terminally differentiate into cell types of the tissue from which they are obtained.

A totipotent cell is defined as a cell which has the potential to develop into an entire organism.

### Embryonic stem cells

Embryonic stem (ES) cells are stem cells derived from the pluripotent inner cell mass (ICM) cells of the pre-implantation, blastocyst-stage embryo. Outgrowth cultures of blastocysts give rise to different types of colonies of cells, some of which have an undifferentiated phenotype. If these undifferentiated cells are sub-cultured onto feeder layers they can be expanded to form established ES cell lines that seem immortal. These pluripotent stem cells can differentiate in vitro into a wide variety of cell types representative the three primary germ layers in the embryo. Methods for deriving ES cells are known for example from Evans et al. 1981; Nature; 29; 154-156.

Methods for isolating human pluripotent stem cells are described, for example, by Trounson, A. O. Reprod. Fertil. Dev 2001; 13 (7-8): 523-32. Isolation requires feeder cells (and 20% fetal calf serum) or conditioned medium from feeder cells. Further methods for producing pluripotent cells are known from WO 01/30978 where the derivation of pluripotent cells from oocytes containing DNA of all male or female origin is described. In addition, stem cell-like lines may be produced by cross species nuclear transplantation as described, for example, in WO 01/19777, by cytoplasmic transfer to de-differentiate recipient cells as described, for example, in WO 01/00650 or by "reprogramming" cells for enhanced differentiation capacity using pluripotent stem cells (see WO 02/14469).

### Adult stem cells

The stem cell used in the present invention is an adult stem cell. An adult stem cell is an undifferentiated cell found among differentiated cells in a tissue or organ, can renew itself, and can differentiate to yield the major specialized cell types of the tissue or organ.

Adult stem cells include mesenchymal, haematopoeitic, neural and epithelial cells. The stem cells are suitably murine, human, porcine, primate, feline or canine although any mammalian stem cells may be used. Preferably the adult stem cell is a neural stem cell.

Preferably the adult stem cells are multipotent.

### Neural stem cells

A neural stem cell is a stem cell found in adult neural tissue and may give rise to neurons, astrocytes, and oligodendrocytes. A review of neural stem cells is given in Galli et al., Circulation Research 92 (6):598; Gage FH. Science. 2000; 287: 1433-1438.

It has recently been shown that cell turnover, including neurons, occurs in the mature CNS, due to the persistence of precursor cells that possess the functional characteristics of bona-fide neural stem cells (NSCs) within restricted brain areas. Stem cells can be isolated from both the subventricular zone (SVZ), a thin layer of dividing cells that lies along the length of the lateral wall of the lateral ventricles, and the hippocampus, a cortical structure in the medial portion of the temporal lobe. Indeed, in the adult mammalian brain, the genesis of new neurons has been consistently documented in the subgranular layer of the dentate gyrus of the hippocampus and the subventricular zone (SVZ) of the lateral ventricles (Galli et al., Circulation Research 92 (6):598; Gage FH. Science. 2000; 287: 1433-1438; Luskin MB. Neuron. 1993; 11; 173-189; Lois C, Alvarez-Buylla A. Science. 1994; 264: 1145-1148).

The SVZ contains four main cell types: newly generated neurons, astrocytes, rapidly dividing precursors, and ependymal cells. The rapidly dividing immature precursors are closely associated with the chains of newly generated neurons that migrate through the glial tubes formed by the processes of SVZ astrocytes. They are scattered in focal clusters along the network of chains. The multiciliated ependymal cells line the ventricular cavity. A series of observations indicate that a specific subtype of SVZ astroglial cells is the actual neural stem cell (Alvarez-Buylla A., J Neurosci. 2002; 22: 629-34).

It has been shown that NSCs can be isolated and grown in vitro from non-canonical neurogenic periventricular regions, in which the mature parenchyma is directly in contact with the ependymal monolayer, such as the fourth ventricle or the spinal cord (Johansson et al., Cell. 1999; 96: 25-34).

The lack of specific markers has hampered the univocal identification and characterisation of NSCs, although, recently, putative stem cell markers AC133, PNA^{low}/HSA^{low}, and LeX/ssea-1 were identified (Uchida et al., Proc Natl Acad Sci U S A. 2000; 97: 14720-14725; Rietze et al., Nature. 2001; 412: 736-739; Capela et al., Neuron. 2002; 35: 865-875).

The use of specific systems has permitted the isolation and expansion of NSCs ex vivo, and has allowed the establishment of NSC lines from various species, including humans (Gage, Science. 2000; 287: 1433-1438; McKay, Science. 1997; 276; 66-71; Gritti et al., Cultures of stem cells of the central nervous system. Chapter 14. In: Fedoroff S, Richardson A, eds. Protocols for Neural Cell Culture. 3rd ed. Totowa, NJ: Humana Press; 2001).

Under appropriate conditions and in the presence of mitogens (epidermal growth factor (EGF) and/or fibroblast growth factor [FGF]), it is possible to induce the proliferation of rapidly dividing precursors from certain neurogenic areas of the adult mammalian brain, for example, the stratum, SVZ, hippocampus and olfactory bulb (Gritti et al., J Neurosci 19, 3287-97 (1999); Gritti et al., J Neurosci 16, 1091-100 (1996)). At least in vitro, these precursors fulfill most of the criteria of bona fide stem cells (Gritti et al., J Physiol Paris. 2002; 96: 81-90; Vescovi et al., Brain Pathol. 1999; 9: 569-598; Weiss et al., Trends Neurosci. 1996; 19: 387-393). On removal of the mitogens, the progeny of NSCs promptly differentiate into the three main cell types of the CNS (astrocytes, oligodendrocytes, and neurons) (McKay R. Science (1997) 276: 66-71).

The most important concept regarding this method is that it represents a selective system by which, in a heterogeneous primary culture, the more committed progenitors and/or differentiated mature cells rapidly die and thus are eliminated, whereas the undifferentiated NSCs are positively selected and forced to access a state of active proliferation. NSCs start proliferating initially as adherent cells and attach to each other, eventually giving rise to spherical clusters that float in suspension and form the so-called "neurospheres". In giving rise to neurospheres, NSCs undergo multiple symmetric cell divisions by which two new NSCs are generated at each cycle. Not all the NSC progeny found in a neurosphere are stem cells. Indeed, only 10% to 50% of these progeny retain stem cell features, whereas the remainders are cells that undergo spontaneous differentiation. Consequently, a neurosphere is a mixture of neural stem cells, transit-amplifying neural progenitors, differentiating progenitors, and even differentiated neurons and glia, depending on the neurosphere size and time in culture. This is the reason why neurospheres are subcultured by harvesting, followed by mechanical dissociation and by re-plating under the same growth conditions. As in the primary culture, differentiating/differentiated cells rapidly die while the NSCs continue to proliferate, giving rise to many secondary spheres and exponential growth in vitro. In this way, stable though heterogeneous NSCs cell lines can be obtained (Galli et al., Circ. Res 92, 598-608 (2003)). Due to these properties, it has been possible to establish continuous mouse transgenic/genetically modified (Galli et al., Development. 2002; 129: 1633-1644) or human NSC lines (Carpenter et al., Exp Neurol. 1999; 158: 265-278; Vescovi et al., Exp Neurol. 1999; 156: 71-83).

Thus it will be appreciated that the term "aNSC" within the scope of the present invention includes adult neural precursor cell (aNPC). It may also include a mixed population of cells, derivable for example from the neurosphere, and including at least one of aNSCs and aNPCs or a combination thereof.

### Inflammation and chronic central nervous system disorders

As defined herein, CNS disorders include any disease or disorder associated with the CNS. CNS disorders encompass numerous acute and chronic afflictions which are characterized either by primary inflammation that leads to secondary neurodegeneration (for example, Multiple Sclerosis (MS), spinal cord injury (SCI), brain trauma and stroke), or by the primary neurodegeneration that is accompanied by secondary reactive inflammation (for example, Parkinson's Disease (PD), Alzheimer's Disease (AD), Huntington's Disease (HD) and epilepsy). The final net effect of such disorders is the loss and/or degeneration of neural cells in particular locations of the CNS, leading to the inability of these cells or the brain region to carry out their intended function which, in turn, causes behaviour abnormalities.

Inflammation is a self-defensive reaction aimed at eliminating or neutralising injurious stimuli, and restoring tissue integrity. Like peripheral inflammation, neuroinflammation can become a harmful process, and it is now widely accepted that it may contributes to the pathogenesis of CNS disorders. CNS inflammation is commonly associated with some degree of tissue damage including, loss of myelin sheaths or loss of axons. For example, such loss is a central theme in human patients with MS. In MS, the immune system attacks the white matter of the brain and spinal cord, leading to disability and/or paralysis. Myelin, oligodendrocytes and neurons are lost due to the release by immune cells of cytotoxic cytokines, autoantibodies and toxic amounts of the excitatory neurotransmitter glutamate.

CNS inflammation (neuroinflammation) has emerged as a factor orchestrating both accumulation, persistence as well as therapeutic efficacy displayed by aNSCs upon systemic injection in subjects suffering from CNS disorders.

Most of our current knowledge about neuroinflammation has been obtained from EAE, which has served a good animal model to study the aspects of CNS inflammation and demyelination seen in human patients with MS. MS and EAE are associated with inflammatory, delayed-type hypersensitivity (Th1) responses, with cytokines playing a prominent role as mediators of intercellular signaling and effector function. Cell adhesion molecules, costimulator ligands and matrix metalloproteinases are also upregulated during the autoimmune response, and are implicated in leukocyte infiltration to the CNS. The inflammatory responses that arise as a result of tissue injury can contribute to further injury (Owens et al., Nature Medicine 7 161-166 (2001)).

Research has shown that EAE and MS are characterised by elevated expression of Th1 and Th1-associated cytokines, chemokines and reactive mediators in the CNS. Cellular sources of these mediators include infiltrating T cells and macrophages, and reactive glia. Expression of pathology-associated molecules tends to diminish in remission or recovery and to reappear in relapse.

A number of cytokines, chemokines and related mediators that have been implicated in EAE. Notably, the Th1-associated cytokines IFN-γ, TNF-α, IL-6 and the Th-1-inducing, cytokine IL-12 are associated with active disease or relapse, whereas the Th-2 cytokines IL-4, IL-10 and IL-13 are associated either with remission or suppression of the disease (Owens et al., Nature Medicine 7 161-166 (2001)).

Detection methods such as, but not limited to magnetic resonance imaging (MRI)-based techniques may significantly help into assessing timing and characteristics of CNS (brain and spinal cord) inflammation, thus allowing the identification of a tight narrow inflammation-related time window for neural stem cell administration. The technique is highly sensitive for the definition of inflammation in MS, stroke, head and spinal cord trauma, and brain tumours. MRI has the ability to detect multifocal white and grey matter lesions, diffuse (occult) disease, and macroscopic tissue atrophy in vivo (Bakshi et al., Neurology 63, S3-11 (2004)). Moreover, contrast-enhanced MRI allows early depiction of active demyelinating lesions in patients suffering from MS, and permits the differentiation of old gliotic lesions from the inflamed new or active ones. Triple dose of gadolinium may enable better assessment of the presence and extent of even "low-grade" MS inflammation (Gasperini, C. et al. Magn Reson Imaging 18, 761-3 (2000)). The employment of novel MRI techniques such as *Diffusion Weighted Imaging* and *Perfusion* allows the identification of both hypoxic and anoxic brain regions during the hyperacute phases of stroke, thus allowing the characterisation of main early (inflammatory) and late (degenerative) stages after cerebral ischaemia (Provenzale, et al., Radiology 229, 347-59 (2003)). Further, MRI allows depiction, assessment of extension, and characterisation and grading of brain tumours (Henson et al., Lancet Oncol 6, 167-75 (2005)).

More generally the present invention may be used to treat primary inflammatory disorders leading to neurodegeneration or primary neurodegenerative disorders in which secondary reactive inflammation is a common accompanying feature. Those diseases are conditions which affect brain function. Neurodegeneration results from deterioration of neurons and/or glia. They are divided into two groups:
- conditions causing problems with movements
- conditions affecting memory and conditions related to dementia.

Thus, the diseases which may be treated by the present invention include the following:
Alexander disease
Alper's disease
Alzheimer disease
Amyotrophic lateral sclerosis
Ataxia telangiectasia
Batten disease (also known as Spielmeyer-Vogt-Sjogren-Batten disease)
Canavan disease
Cockayne syndrome
Huntington disease
Kennedy's disease
Krabbe disease
Lewy body dementia
Metakromatic leukodistrophy
Multiple sclerosis
Multiple System Atrophy
Parkinson disease
Pelizaeus-Merzbacher Disease
Pick's disease
Primary lateral sclerosis
Refsum's disease
Sandhoff disease
Tay-Sachs disease

Inflammatory disorders are characterized by their systemic effects. The immune response in these illnesses may cause dysfunction in tissues other than the typically affected organs. When the CNS is involved, a wide range of neurologic symptoms occurs, including epileptic seizures as well as headaches, confusion, and coma. Seizures or other neurologic abnormalities sometimes may be the initial or even the only manifestation of a systemic inflammatory disorder.

The present disclosure may also be useful in the treatment of systemic or organ specific disorders characterised by chronic inflammation, such as rheumatoid arthritis and type 1 diabetes.

### Cell surface adhesion molecules

Evolution of inflammatory and immune reactions is dependent upon the recruitment and migration of circulating leukocytes to sites of injury or antigen deposition. The accumulation of leukocytes is dependent not only on chemotactic signals emanating from the inflammatory site, but also on cell-cell and cell-matrix interactions. Many of these cellular and matrix interactions are dependent upon expression of cell surface adhesion molecules (CAMs) [integrins, cell surface proteoglycans, selectins, etc.] which facilitate targeting and retention of circulating cells to sites of immunologic challenge (Springer, Nature, 346: 425-434 (1990); Albeda et al., FASEB J., 4: 2668-2680 (1990); Ruoslahti, J. Clin. Invest., 87: 1-5 (1991)).

Integrins represent a family of cell surface heterodimeric proteins that mediate cell adhesion to other cells and to extracellular matrix constituents, including fibronectin.

Although the role of integrins and other CAMs in mediating arrest and adhesion of inflammatory cells prior to extravasation is complex, evidence suggests that integrins may be pivotal in these events.

At a cellular and molecular level, SVZ-derived aNSCs express α4 integrin very late antigen (VLA)-4, and CD44 a finding similar to that described in immune cells. aNSCs spontaneously adhere to purified vascular cell adhesion molecule (VCAM)-1, the VLA-4 counter-ligand. Indeed, aNSCs selectively enter the inflamed CNS through constitutively activated integrins such as VLA-4 and/or functional chemokine receptors such as CCR2, CCR5, CXCR3 and CXCR4.

Thus, the aNSCs used in the present invention may use GPCRs along with CAMs, to further improve their migratory efficacy toward CNS inflamed lesions.

The stem cells used in the present invention may be genetically modified. For, example, they may be modified to express a cell surface adhesion molecule as described above.

Genetic modification may be achieved by introducing into the stem cells vectors and polynucleotides encoding the gene of interest. Preferably the vector and/or polynucleotide expresses the gene of interest.

### Major death receptor ligands

Death receptor ligands, such as Fas ligand (FasL) and TNF-related apoptosis-inducing ligand (TRAIL) are able to induce apoptosis by binding to their cell membrane receptors. Recombinant forms of these ligands are capable to potentiate the effect of chemotherapeutic drugs in vitro and in vivo in the animal model (see Jong et al., Cancer Metastasis Rev. 2001;20(1-2):51-6).

### Vectors

As it is well known in the art, a vector is a tool that allows or facilitates the transfer of an entity from one environment to another. In accordance with the present invention, and by way of example, some vectors used in recombinant DNA techniques allow entities, such as a segment of DNA to be transferred into a host and/or a target cell for the purpose of replicating the vectors comprising the nucleotide sequences used in the invention and/or expressing the proteins used in the invention. Examples of vectors used in recombinant DNA techniques include but are not limited to plasmids, chromosomes, artificial chromosomes or viruses.

Polynucleotides used in the invention are preferably incorporated into a vector.

Preferably, a polynucleotide in a vector for use in the invention is operably linked to a control sequence that is capable of providing for the expression of the coding sequence by the host cell, i.e. the vector is an expression vector. The term "operably linked" means that the components described are in a relationship permitting them to function in their intended manner. A regulatory sequence "operably linked" to a coding sequence is ligated in such a way that expression of the coding sequence is achieved under conditions compatible with the control sequences.

The control sequences may be modified, for example by the addition of further transcriptional regulatory elements to make the level of transcription directed by the control sequences more responsive to transcriptional modulators.

The vectors used in the present invention may be for example, plasmid or virus vectors provided with an origin of replication, optionally a promoter for the expression of a polynucleotide and optionally a regulator of the promoter. The vectors may contain one or more selectable marker genes, and/or a traceable marker such as GFP. Vectors may be used, for example, to transfect or transform a host cell.

Control sequences operably linked to sequences encoding proteins for use in the invention include promoters/enhancers and other expression regulation signals. These control sequences may be selected to be compatible with the host cell for which the expression vector is designed to be used in. The term "promoter" is well-known in the art and encompasses nucleic acid regions ranging in size and complexity from minimal promoters to promoters including upstream elements and enhancers.

The promoter is typically selected from promoters which are functional in mammalian cells, although prokaryotic promoters and promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of α-actin, β-actin, tubulin) or, alternatively, a tissue-specific manner (such as promoters of the genes for pyruvate kinase). Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

### Protein

As used herein, the term "protein" includes single-chain polypeptide molecules as well as multiple-polypeptide complexes where individual constituent polypeptides are linked by covalent or non-covalent means. As used herein, the terms "polypeptide" and "peptide" refer to a polymer in which the monomers are amino acids and are joined together through peptide or disulfide bonds. The terms subunit and domain may also refer to polypeptides and peptides having biological function.

### Polynucleotides

Polynucleotides used in the invention may comprise DNA or RNA. They may be single-stranded or double-stranded. It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides used in the invention to reflect the codon usage of any particular host organism in which the polypeptides are to be expressed. The polynucleotides may be modified by any method available in the art. Such modifications may be carried out in order to enhance the *in vivo* activity or life span of polynucleotides of the invention.

### Variants, Derivatives, Analogues, Homologues and Fragments

In addition to the proteins and nucleotides mentioned herein, the present invention also encompasses the use of variants, derivatives, analogues, homologues and fragments thereof.

In the context of the present invention, a variant of any given sequence is a sequence in which the specific sequence of residues (whether amino acid or nucleic acid residues) has been modified in such a manner that the polypeptide or polynucleotide in question retains at least one of its endogenous functions. A variant sequence can be obtained by addition, deletion, substitution modification replacement and/or variation of at least one residue present in the naturally-occurring protein.

The term "derivative" as used herein, in relation to proteins or polypeptides of the present invention includes any substitution of, variation of, modification of, replacement of, deletion of and/or addition of one (or more) amino acid residues from or to the sequence providing that the resultant protein or polypeptide retains at least one of its endogenous functions.

The term "analogue" as used herein, in relation to polypeptides or polynucleotides includes any mimetic, that is, a chemical compound that possesses at least one of the endogenous functions of the polypeptides or polynucleotides which it mimics.

### Treatment

It is to be appreciated that all references herein to treatment include curative, palliative and prophylactic treatment. The treatment of mammals is particularly preferred. Both human and veterinary treatments are within the scope of the present invention.

In a preferred embodiment, treatment is given after the onset of a CNS disorder.

### Pharmaceutical compositions

The stem cells used in the present invention may be in the form of a pharmaceutical composition. A pharmaceutical composition is a composition that comprises or consists of a therapeutically effective amount of a pharmaceutically active agent. It preferably includes a pharmaceutically acceptable carrier, diluent or excipients (including combinations thereof). Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Examples of pharmaceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

### Administration

The stem cells used in the present invention may be administered by any suitable method. Such methods are well known to persons skilled in the art. In one embodiment, the stem cells are administered parentally, for example intracavernosally, intravenously, into the cerebrospinal fluid (intrathecally and/or intracisternaly), intramuscularly or subcutaneously. For parenteral administration, the compositions may be best used in the form of a sterile aqueous solution which may contain other substances, for example enough salts or monosaccharides to make the solution isotonic with blood. The stem cells may be administered directly to the location in which the stem cells reside in the body. Preferably, the aNSCs used in the present invention are administered intravenously or intrathecally.

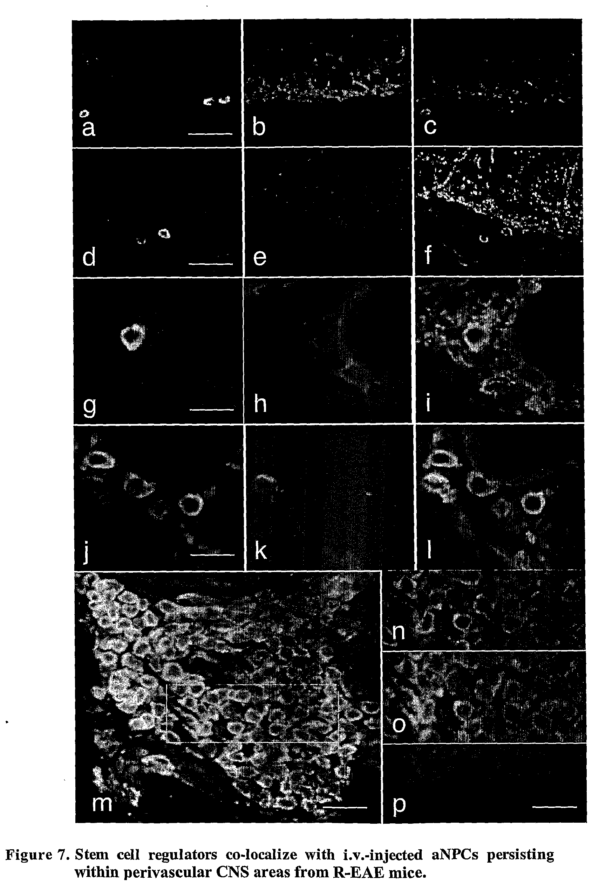

Further preferred features and embodiments of the present invention will now be described by way of non-limiting example and with reference to the accompanying drawings in which:

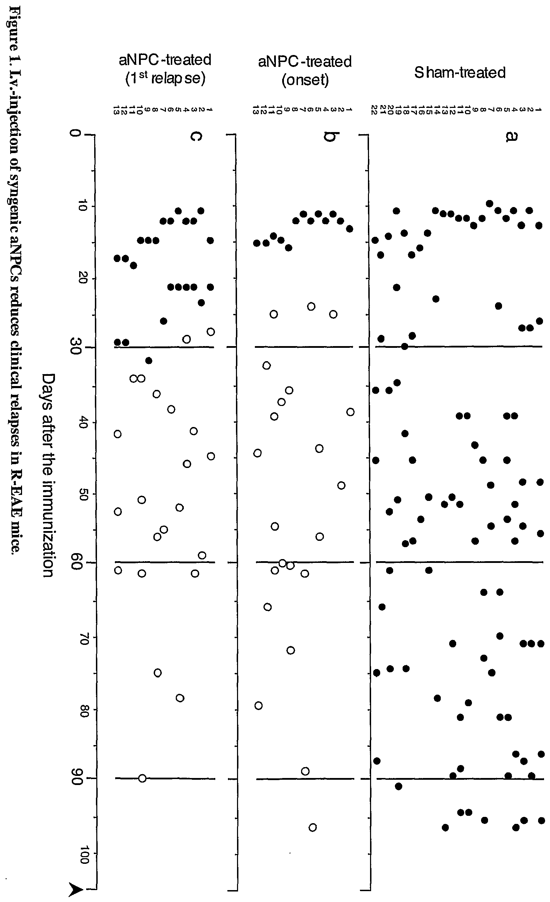

**Figure 1****. I.v.-injection of syngenic aNPCs reduces clinical relapses in R-EAE mice.**
   Graphic visualization of clinical relapse occurrence (each dot represents a single relapse) in both sham- (**a**) and aNPC-treated (**b** and **c**) R-EAE mice. Black dots represent single relapses occurred during an off-therapy period while white dots represent relapses occurring during aNPC-treatment. Each mouse is represented with a number on the y-axis. Syngenic aNPCs were transplanted i.v. at either disease onset (**b**) or at the 1^{st} clinical relapse (**c**). All mice have been sacrificed 106 dpi (black arrowhead) during clinical remission. Two-fold less clinical relapses were recorded in R-EAE mice transplanted at disease onset as early as between 30 and 60 dpi; they maintained a similarly low relapse rate up to the end of the follow-up. R-EAE mice transplanted at 1^{st} relapse recovered later, but showed a dramatic 3-fold reduction of the relapse rate between 60 and 90 dpi.

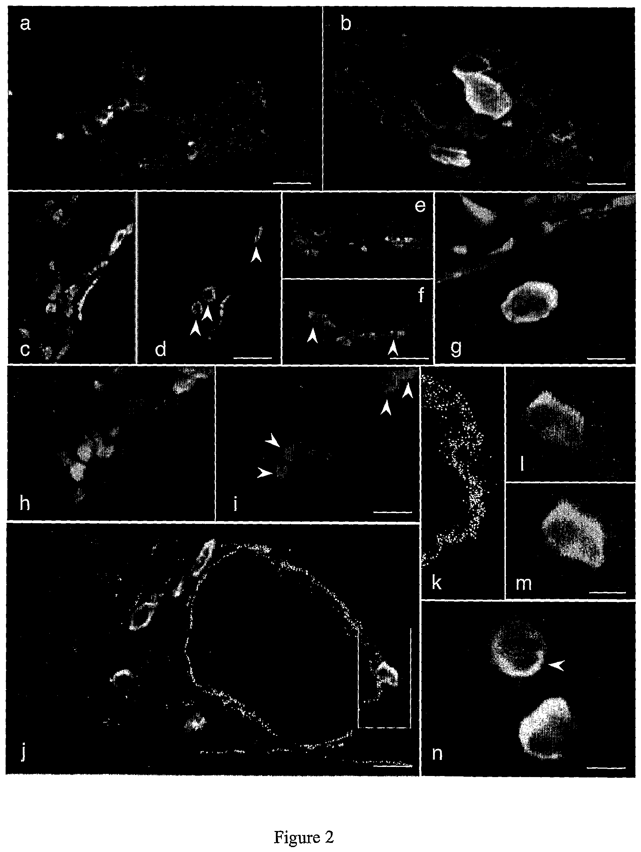

**Figure 2****. I.v.-transplanted aNPCs persist within perivascular inflamed CNS areas from R-EAE mice up to 3 months post-transplantation.**
   **a, b** β-gal-labeled i.v-injected aNPCs (lighter) remain within perivascular CNS areas where blood-borne CD45⁺ immune cells (**b**) - forming the CNS inflammatory infiltrates - persist. CD45⁺ cells within perivascular areas are Ki67⁻ (**b**), thus indicating that they are final effector encephalitogenic cells. Nuclei in **a** are in darker shade (Dapi). Scale bar: **a**, 30 µm; **b**, 15 µm.
   **c-m,** Within perivascular areas, some of the β-gal-labeled cells (lighter shade in **c**, **e**, **g**, **h**, **j**, **l**) are positive for nestin (**d**, arrowheads), NeuN (**f**, arrowheads), Mash-I (**g**), DIx-2 (**i**, arrowheads), and PSA-NCAM (**j**, **m**). In **j** and **k** CNS blood vessels are stained with laminin. The inset in **j** is shown in **k-m.** Nuclei in **c**, **e**, **h** and **j** are stained with Dapi. Scale bars: **d**, 30 µm; **f**, 25 µm; **g**, 15 µm; **i**, 40 µm; **j**, 20 µm; **m**, 10 µm.
   **n,** A β-gal-labeled i.v.-injected aNPC stained for BrdU (arrowhead). Scale bar, 10 µm.

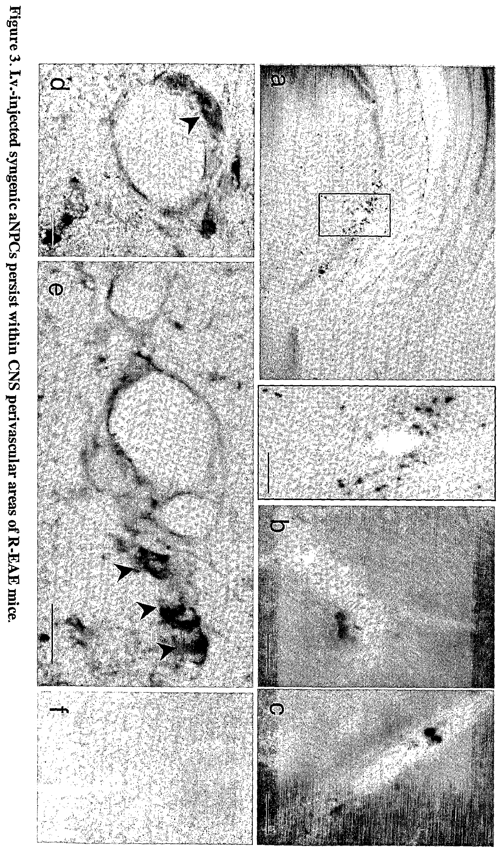

**Figure 3****. I.v.-injected syngenic aNPCs persist within CNS perivascular areas of R-EAE mice.**
   **a-c** X-gal staining of vibratome-cut (70 µm) brain and spinal cord tissue sections from R-EAE mice injected i.v. with syngenic aNPCs, showing transplanted X-gal⁺ cells (black cells) persisting within perivascular CNS areas up to the end of the clinical follow-up (106 dpi). Scale bars: **a**, 80 µm; **b** and **c**, 40 µm. **d** X-gal⁺ i.v.-injected aNPCs (black) persisting within perivascular CNS areas, retain nestin⁺ (arrowhead) phenotype. Scale bar, 20 µm. **e** Few migrating aNPCs express NeuN⁺ (arrowheads) as long as they move out of the perivascular area. Scale bar, 35 µm. **f**, X-gal staining in a representative spinal cord section from a sham-treated R-EAE mouse (20X magnification).

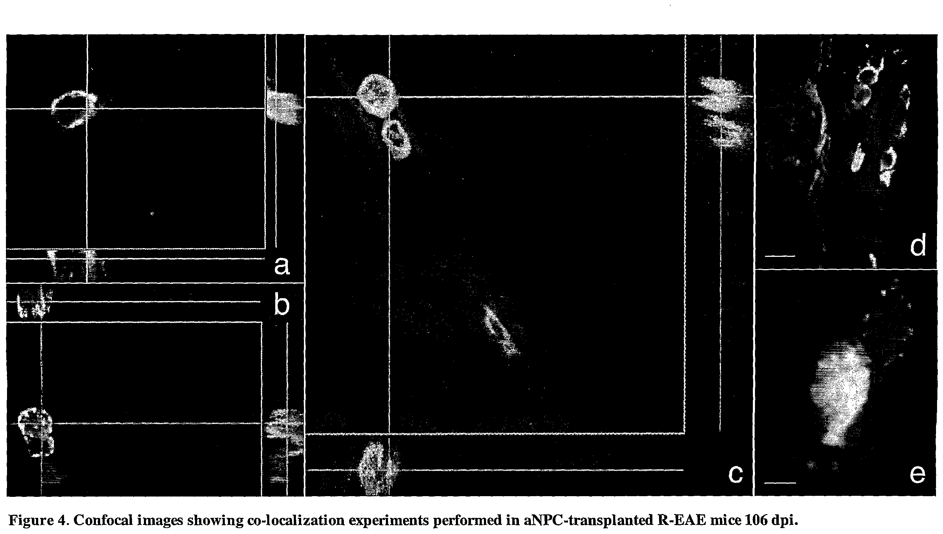

**Figure 4****. Confocal images showing co-localization experiments performed in aNPC-transplanted R-EAE mice 106 dpi.**
   **a**, Proliferating (BrdU) i.v.-transplanted β-gal⁺ cell expressing PSA-NCAM within a spinal cord perivascular area (100X magnification), **b**, Jagged-1 expression on the membrane of an i.v.-transplanted β-gal⁺ cell (100X magnification), **c**, Two β-gal⁺ (green) i.v.- transplanted proliferating aNPCs, as indicated by incorporation of BrdU (red), still persisting within CNS perivascular area (63X magnification). None of the β-gal⁺ cells were positive for GFAP (**d**, red) or NG2 (**e**). In panels **b**, **d** and **e** cell nuclei are stained with Dapi. Scale bars: **d**, 40 µm; **e**, 20 µm.

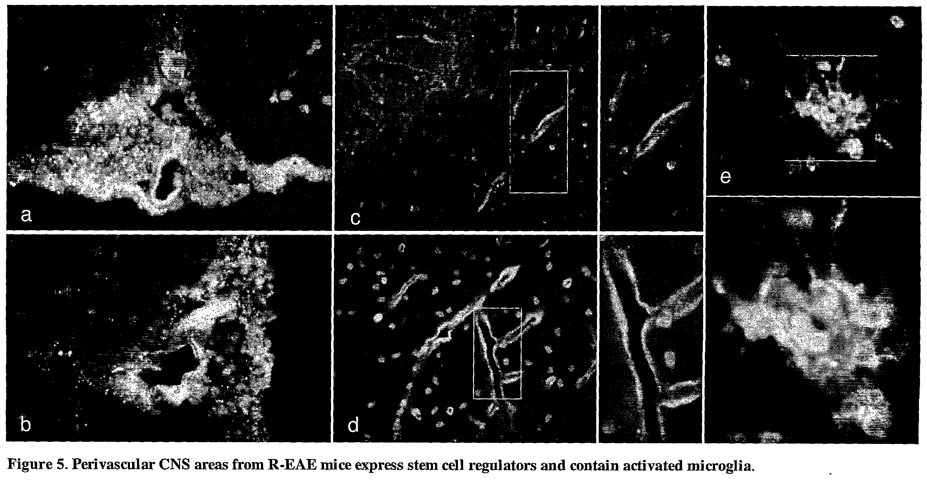

**Figure 5****. Perivascular CNS areas from R-EAE mice express stem cell regulators and contain activated microglia.**
   **a-d**, BMP-4 (**a** and **b**) and Noggin (**c** and **d**) expression within perivascular areas of the CNS from sham-treated R-EAE mice. Inflammatory blood-borne mononuclear cells are visible within these areas. Blood vessels are stained with anti-CD31/PECAM antibody (X40 magnification). **e**, Ramified activated CD11b⁺ microglial cells in a spinal cord tissue section from a representative aNPC-transplanted R-EAE mouse (X40 magnification). Activated microglial cells were found intermingled with CNS-infiltrating blood-borne immune cells. In all panels, cell nuclei are stained with Dapi.

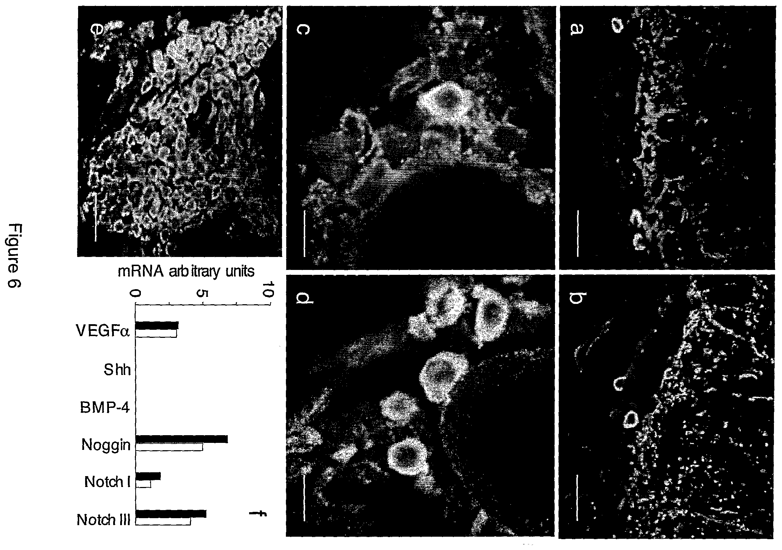

**Figure 6****. Stem cell regulators co-localize with i.v.-injected aNPCs persisting within perivascular CNS areas from R-EAE mice.**
   **a-e**, In perivascular CNS areas, GFAP⁺ astrocytes (**a** and **b**) and laminin⁺ endothelial cells (**c** and **d**) - secreting BMP-4 (**a** and **c**) and Noggin (**b** and **d**) - co-localize with transplanted β-gal⁺ aNPCs (**a**-**e**). **e**, Perivascular CNS area containing infiltrating final effector CD45⁺ lymphocytes secreting Noggin. Nuclei are stained with Dapi. Scale bars: **a** and **b**, 40 µm; **c** and **d**, 25 µm; **e**, 50 µm. Single staining are available in Figure 7.
   **f**, Real-time PCR showing mRNA levels of stem cell regulators in spleen-derived lymphocytes from SJL naive mice. Both Con A-activated (black bars) and resting lymphocytes (white bars) produce VEGF-α, Noggin, Notch I and III.
**Figure 7****. Stem cell regulators co-localize with i.v.-injected aNPCs persisting within perivascular CNS areas from R-EAE mice.**
   **a-l**, GFAP⁺ astrocytes (**b** and **e**) and laminin⁺ endothelial cells (**h** and **k**) - secreting BMP-4 (**c** and **i**) and Noggin (**f** and **l**) - co-localize with transplanted β-Gal⁺ aNPCs (**a**, **d**, **g**, **j**). Scale bars: **a** and **c**, 80 µm; **g** and **j**, 40 µm. **m-p**, Merged confocal image (**m**) of a representative perivascular CNS area containing infiltrating .final effector CD45⁺ lymphocytes (**n**) secreting Noggin (**o**). Cell nuclei are stained with Dapi (**p**). Scale bars: **m**, 30 µm; **n-p** 20 µm.

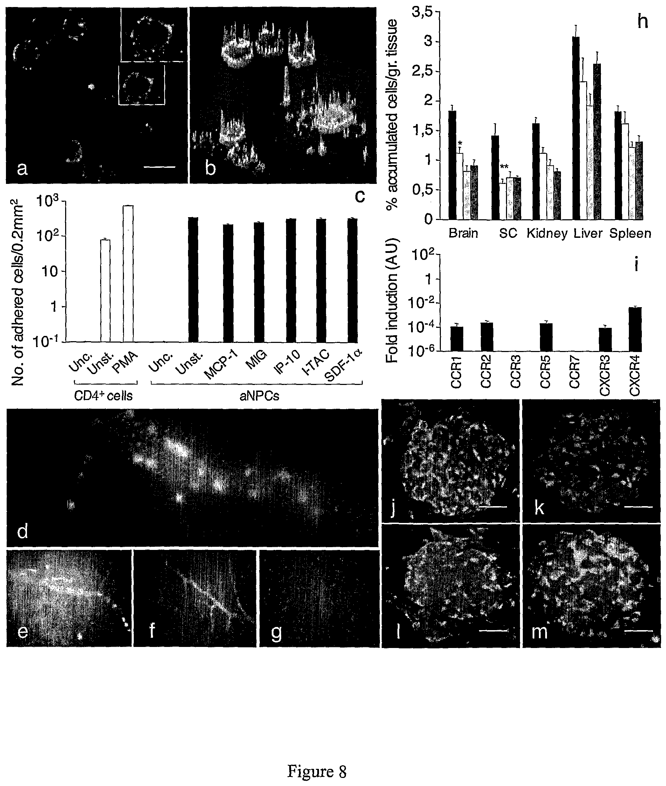

**Figure 8****. aNPCs constitutively expressing VLA-4 and chemokine receptors accumulate around inflamed CNS microvessels from R-EAE mice.**
   **a, b** Clusterization (**a**) and 3D plot of fluorescence intensity (**b**) of VLA-4 on aNPCs. Nuclei are stained with Dapi. Scale bar, 20 µm.
   **c**, Unstimulated (Unst.) aNPCs and PMA-stimulated CD4⁺ T cells adhere to VCAM-1 in vitro. Binding does not increase upon aNPC stimulation (for 3 min. at 37°C) with 1µM of different chemokines. Adhesion is completely absent in uncoated (Unc.) wells.
   **d-g**, Intravital microscopy showing aNPCs (bright intravascular dots) firmly adhering to inflamed brain (**d**) and striate muscle venules (**e**) from LPS-treated mice (20X magnification). VCAM-1 expression in inflamed striate muscle venules is shown in **f**. Isotype-matched control antibody (anti-human Ras) (**g**).
   **h**, Accumulation of 2,3-[³H]-glycerol-labelled aNPCs - 24 hs after i.v. injection - into different organs from R-EAE (black bars) and naive control (light bars bars) mice. Blocking anti-VLA-4 antibody (PS/2) decreases aNPC recruitment in the brain (39%) and spinal cord (54%) from R-EAE mice (white bars), but not from naive aNPC-injected control mice (dark grey bars) (*p=0.005 and **p≤ 0.0001).
   **i**, Mouse aNPCs express detectable mRNA levels of CCR1, CCR2, CCR5, CXCR3 and CXCR4, but not of CCR3 and CCR7.
   **j-m**, Nestin-positive aNPCs express CCR2 (**j**), CCR5 (**k**), CXCR3 (**l**) and CXCR4 (**m**). Nuclei are stained with Dapi. Scale bars: **j-l**, 80 µm; **m**, 120 µm.

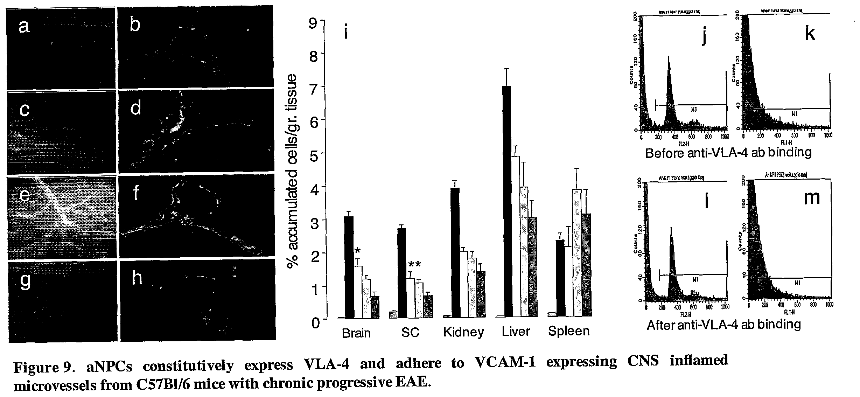

**Figure 9****. aNPCs constitutively express VLA-4 and adhere to VCAM-1 expressing CNS inflamed microvessels from C57BI/6 mice with chronic progressive EAE.**
   **a-h**, VCAM-1 expression on CNS endothelial cells in C57B1/6 EAE mice immunized with MOG35-55. Intravital microscopy (bright dots in **a, c, e, g**) and immunofluorescence (fluorescence in **b, d, f, h**) images showing Alexa 488-labeled anti-VCAM-1 mAb accumulating in cerebral blood vessels at 24 dpi (**e** and **f**). Lower level of VCAM-1 was detectable at earlier (15 dpi, **c** and **d**) and later (35 dpi, **g** and **h**) time points (40X magnification). Blood vessels were stained with anti-laminin. Nuclei were counterstained with Dapi. Magnification: **a**, **c**, **e** and **g**, 20X; **b**, **d**, **f** and **h**, 40X. **i**, Accumulation of 2,3-[³H]-glycerol-labelled aNPCs into different organs from MOG35-55-immunized C57B1/6 mice (black bars) 24 hs after i.v. cell injection. Recruitment of 2 x10⁶ i.v.-injected aNPCs is decreased in the brain (42%) and spinal cord (54%) after in vitro pre-treatment with blocking rat anti-mouse VLA-4 antibodies (white bars) (*p≤ 0.001 and ** p≤ 0.0001). Naive control mice transplanted i.v. with syngenic aNPCs pre-treated (dark grey bars) or not (light grey bars) with blocking rat anti-mouse VLA-4 antibodies, and sham-treated naive mice (mid grey bars) were used as controls. Data are expressed as mean percentage of accumulated cells/gr. tissue (±SE) of at least 6 mice per group from four independent experiments. **j-m**, FACS analysis showing the absence of any necrotic (PI staining in **j** and **l**) or apoptotic (annexin-V staining in **k** and **m**) effect induced by the binding of the anti-VLA-4 blocking antibody on aNPCs.

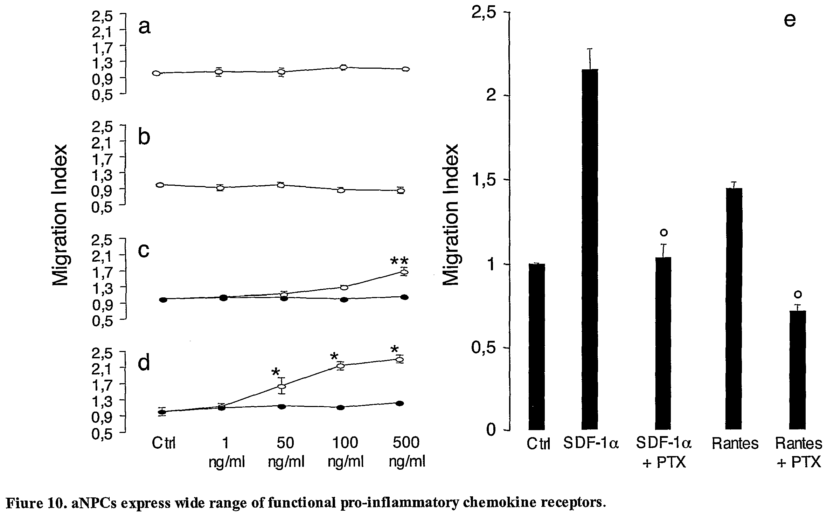

**Figure 10****. aNPCs express wide range of functional pro-inflammatory chemokine receptors.**
   **a-d,** Four hours-chemotaxis assay of mouse aNPCs in response to CCL2/MCP-1 (**a**), CCL3/MIP-1α (**b**), CCL5/Rantes (**c**) and CXCL12/SDF-1α. (**d**). The number of migrating aNPCs significantly increased with SDF-1α and Rantes. White dots in the panels indicate *chemotaxis* experiments, whereas black dots indicate *chemokinesis* experiments. **e**, aNPCs pre-treated with PTX (0.01 mg/ml 15 minutes at r.t.) failed to respond to both CXCL12/SDF-1α and CCL5/Rantes (°p≤ 0.0005; *p≤ 0.005; **p≤ 0.05).

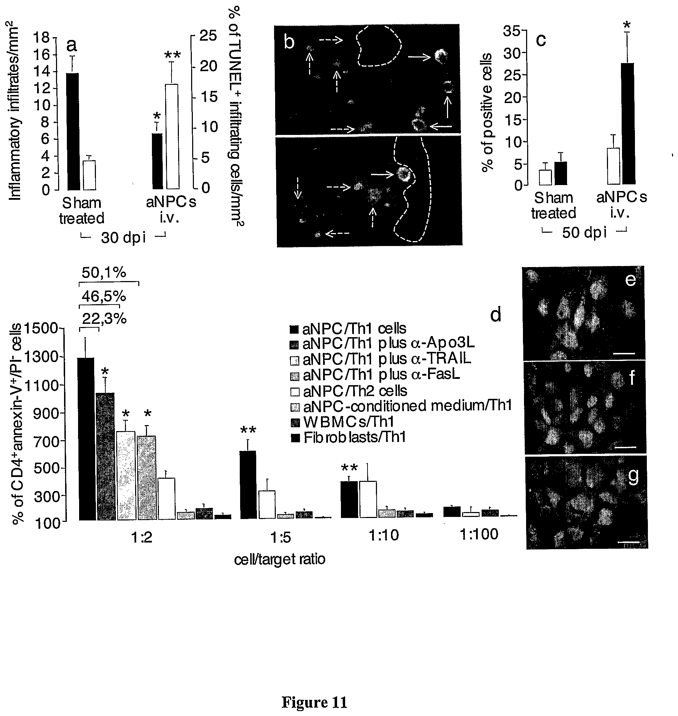

**Figure 11****. aNPCs induce apoptosis of encephalitogenic T cells in vitro and in vivo.**
   **a**, Decrease of spinal cord inflammation (black bars) and increase of CNS-infiltrating apoptotic lymphocytes (white bars) in R-EAE mice 15 days after aNPC injection (30 dpi) (mean ± SE; *p≤ 0.01; **p≤ 0.005, when compared to Sham-treated mice).
   **b** Spinal cord perivascular area double stained for caspase-3 (dashed arrows) and β-gal⁺ (solid arrows). Dashed lines indicate blood vessels (100X magnification).
   **c**, FACS analysis showing late apoptotic (TOPRO3⁺/AnnexinV⁺, black bars) - but not necrotic (TOPRO3⁺/AnnexinV⁻, white bars) - CNS infiltrating CD3⁺ T cells significantly increased in R-EAE mice 35 days after aNPC injection (50 dpi).
   **d**, aNPCs induce apoptosis (AnnexinV⁺/PI⁻ cells) of PLP139-151 Th1, but not Th2, cell lines. Inhibition of FasL, Apo3L, or TRAIL significantly reduces aNPC-mediated pro-apoptotic effect (*p≤ 0.01; **p≤0.05 vs. basal levels).
   **e-g**, Fluorescence images showing expression of death receptors (FasL/CD95- ligand [**e**], Apo3L [**f**], TRAIL [**g**]) on aNPCs conditioned with pro-inflammatory cytokines. Nuclei are stained with Dapi. Scale bars, 15 µm.

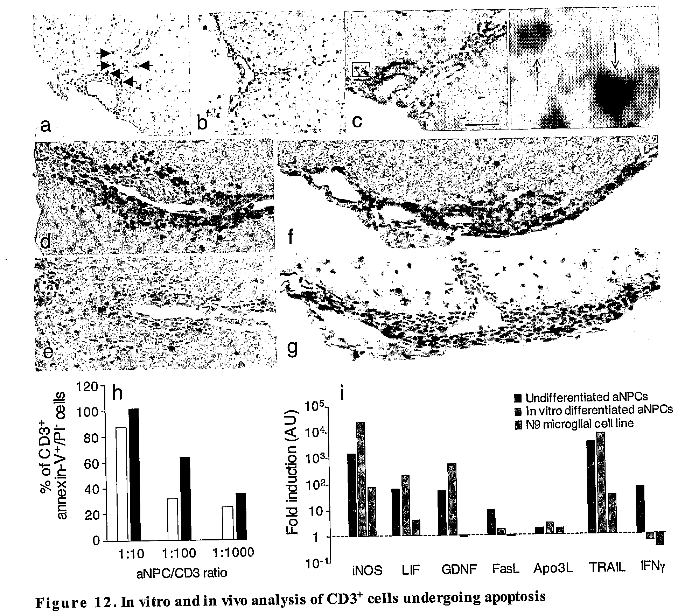

**Figure 12****. In vitro and in vivo analysis of CD3⁺ cells undergoing apoptosis.**
   **a** and **b**, Representative spinal cord perivascular areas stained for TUNEL from either sham- (**a**) or aNPC-treated R-EAE mice (**b**) (20X magnification). Only few apoptotic cells (arrows) are visible in **a** while the great majority of the cells surrounding the blood vessel in **b** are TUNEL⁺ (black dots). **c** Spinal cord perivascular area double stained for TUNEL and CD3 (dashed arrow, TUNEL⁺CD3⁻ cell; solid arrow, TUNEL⁺CD3⁺; Scale bar, 30 µm). **d-g**, Representative consecutive (5 µm-tick) spinal cord sections - stained for CD3 dots in **d** and **f**) or TUNEL (black dots in **d** and **f**) - showing perivascular areas from sham-treated (**d** and **e**) or aNPC-injected (**f** and **g**) R-EAE mice (40X magnification). Nuclei in **d** and **f** have been counterstained with haematoxilin. Note that the great majority of apoptotic cells expressing CD3 - which are significantly increased in aNPC-treated mice (p< 0.005 vs. sham-treated) - are confined within perivascular inflamed CNS areas, as early as 2 weeks p.t. (30 dpi). **h**, CD3/CD28 activated spleen-derived lymphocytes undergo apoptosis (AnnexinV⁺/PI⁻ cells) when co-cultured with aNPCs (single well, black bars; trans-well, white bars). **i**, Pro-inflammatory cytokine-conditioned aNPCs express mRNA of pro-apoptotic molecules. Arbitrary units (AU) represent fold induction of mRNA levels between conditioned and non-conditioned cells.

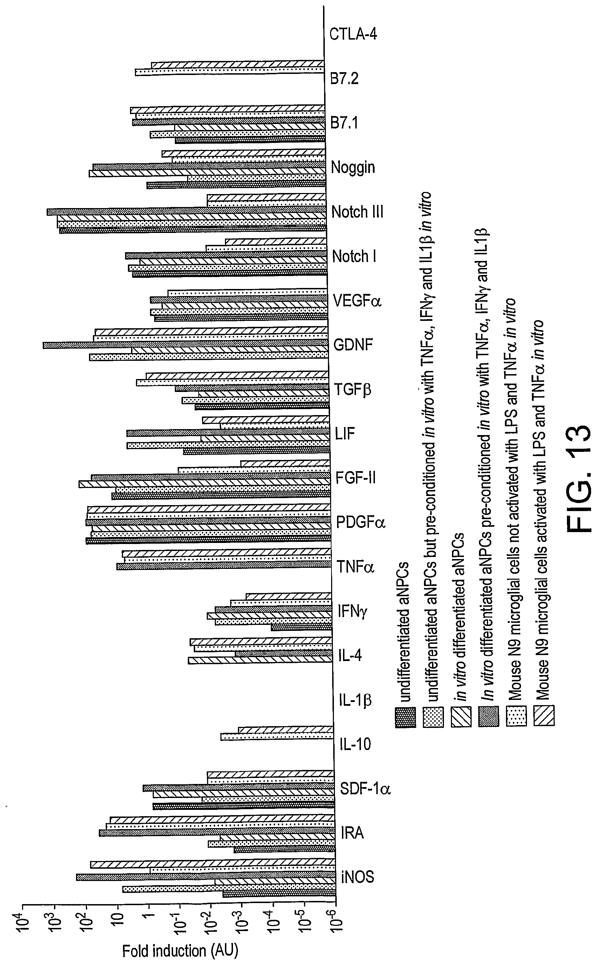

**Figure 13****. Expression of regulators of stem cell proliferation and differentiation, immune molecules and trophic factors by aNPCs.**
   RT-PCR analysis showing mRNA levels of inducible nitric oxide synthase (iNOS), interleukin-1 receptor antagonist (IRA), CXCL12/SDF-1α, interleukin (IL)-10, IL-1β, IL-4, tumor necrosis factor (TNF)α, interferon (IFN)γ, platelet-derived growth factor (PDGF)α, fibroblast growth factor (FGF)-II, leukaemia inhibitory factor (LIF), transforming growth factor (TGF)β, glial derived neurotrophic factor (GDNF), VEGFα, Notch I, Notch III, Noggin, B7.1, B7.2, and cytotoxic T-lymphocyte antigen (CTLA)-4. mRNA levels were measured in undifferentiated aNPCs, in vitro differentiated aNPCs, undifferentiated aNPCs but pre-conditioned with TNFα, IFNγ and IL1β_{.} in vitro, and in vitro differentiated aNPCs pre-conditioned in vitro with TNFα, IPNγ and IL1β. Mouse N9 microglial cells activated or not with LPS and TNFα in vitro were used as controls. Data are express as arbitrary units (AU) and represent fold-induction of mRNA level in the different cell populations over ConA-stimulated spleen-derived lymphocytes. aNPC cell samples were obtained at ≤ 10 passages of amplification in vitro.

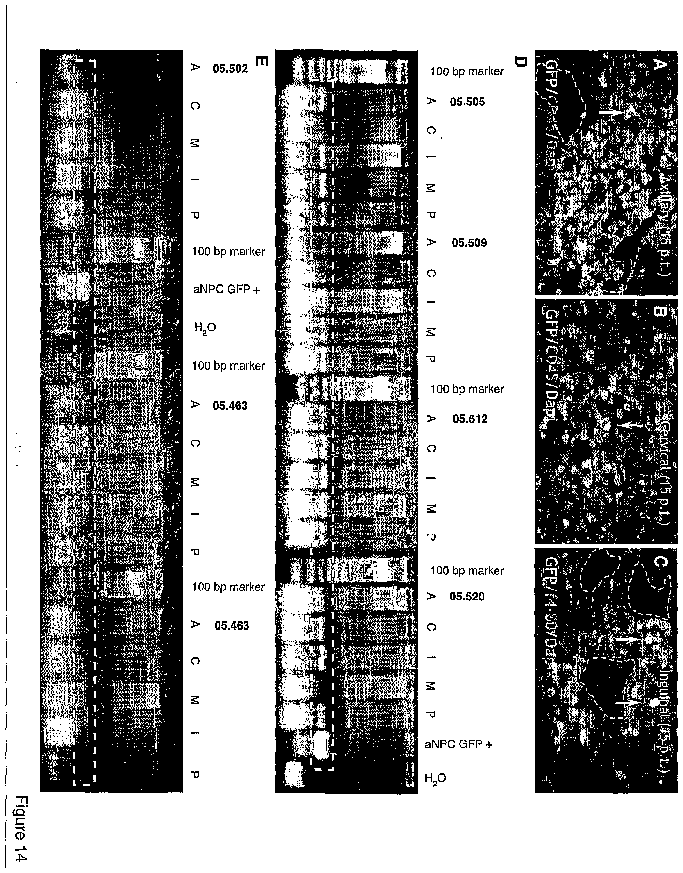

**Figure 14****. Intravenously-injected aNPCs accumulated and persisted over 100 days after transplantation within major secondary lymphoid organs of R-EAE mice.**
   **a-c**, at 15 day after intravenous injection, enhanced green fluorescence protein (eGFP)-immunoreactive transplanted aNPCs persist within the context of lymph node stroma in periduttal areas (dashed line) and do not co-localize with either CD45 (**a** and **b**) or f4-80 (**c**) immune markers. In all panels, cell nuclei are stained with Dapi. **d and e**, One-step real time RT-PCR for eGFP from major draining lymph node stations of R-EAE mice injected intravenously with syngeneic aNPCs and sacrificed at either 15 (**d**) or 100 days after transplantation (**e**). Note the presence of variably intense band of 307 bps for eGFP in most of samples (dashed box in **d** and **e**). Legend: A, axillary, C, cervical, I, inguinal, M, mesenteryc, P, paraortic.

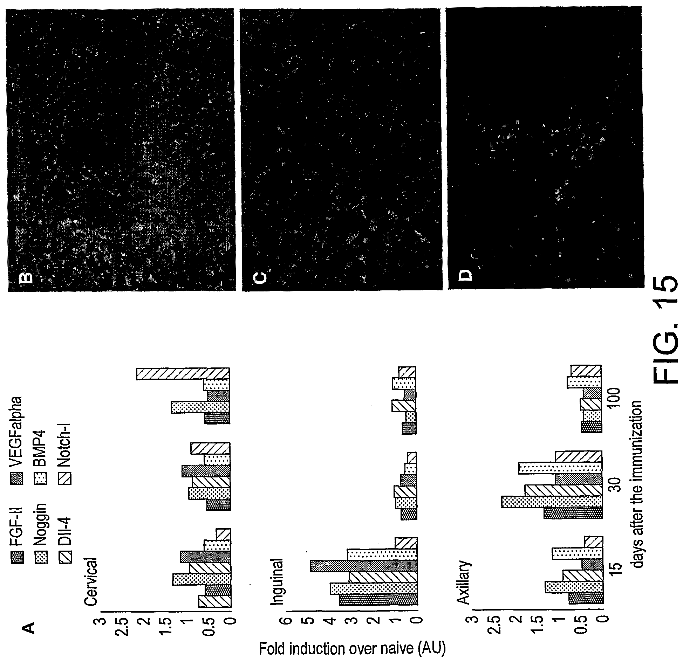

**Figure 15****. Draining lymph node from R-EAE mice dynamically express of major regulators of somatic stem cells at both protein and mRNA levels.**
   **a**, RT-PCR analysis showing mRNA levels of major regulators of somatic stem cells within cervical, inguinal and axillary draining lymph nodes from R-EAE mice at different time points (15, 30 and 100 days) after the active immunization with PLP. Data are express as arbitrary units (AU) and represent fold-induction of mRNA levels in the lymph nodes from R-EAE mice over lymph nodes from naive (sex-, age- and weight-matched) control mice.
   **b-d**, Tenascin C (**b**), sonic hedgehog (**c**) and Jagged-1 (**d**) protein expression within cervical draining lymph nodes from EAE mice.

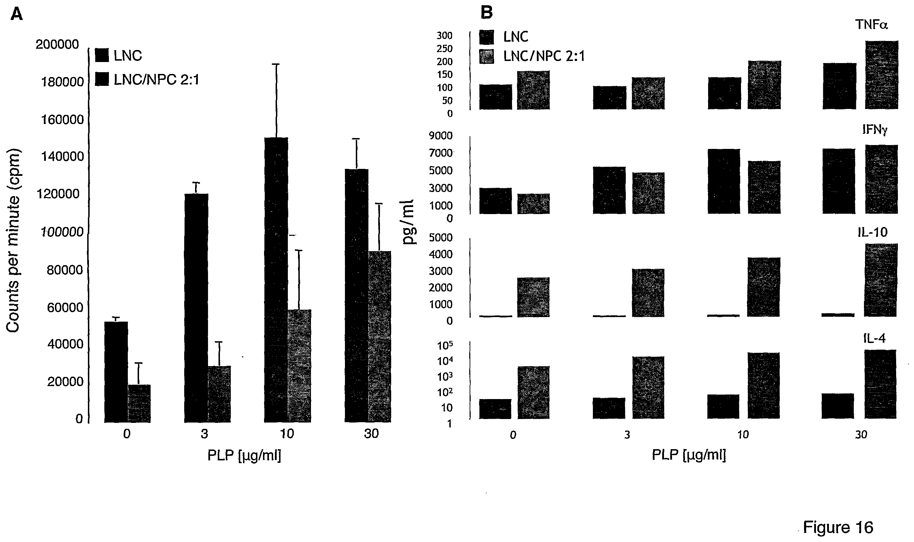

**Figure 16****. aNSCs inhibit antigen-specific proliferation of encephalitogenic CD4⁺ cells.**
   **a**, The effect of aNSC on H-thymidine incorporation in vitro on lymph node cells (LNCs), obtained from R-EAE-induced SJL mice. The effect of co-culturing LNCs with aNSCs were examined in response to 0-30 µg/ml of PLP139-151. aNSCs significantly suppressed 3H-thymidine incorporation in LNCs, as compared to control in a dose dependent manner.
   **b**, Multiple immunochemiluminescence ELISA-based Thl/Th2 cytokine analysis of supernatants collected from LNC/aNSC co-cultures. Th2-like putative anti-inflammatory cytokines (e.g., IL-10, IL-4) are found to be significantly higher on samples from CD4+ T lymphocytes co-cultured with aNSCs, when compared to non co-cultured PLP-reactive CD4+ control cell lines.

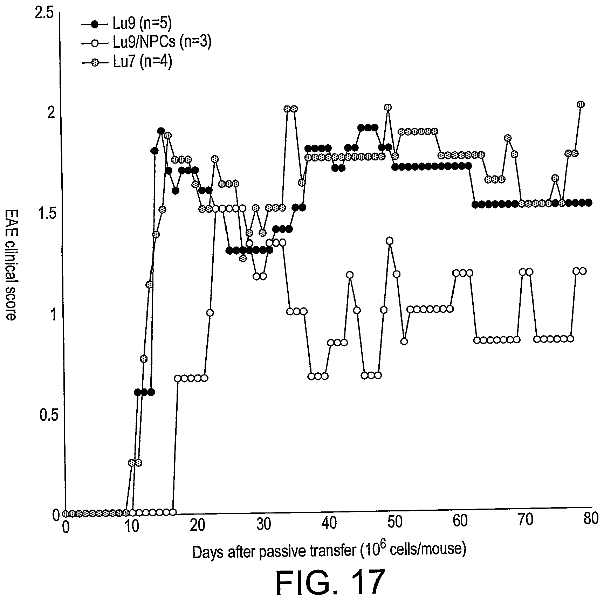

**Figure 17****.** PLP139-151-reactive T cell lines co-cultured with syngeneic aNSCs resulted significantly less encephalitogenic than non co-cultured counterparts, when studied in an EAE adoptive transfer setting in vivo.

### Example 1 - Materials and methods

### Adult NPC derivation and cultures

aNPC cultures were established from the SVZ of the brain of 6-8 week-old SJL and C57BL/6 mice, as previously described (Pluchino S. et al. Injection of adult neurospheres induces recovery in a chronic model of multiple sclerosis. Nature 422, 688-94 (2003)). Briefly, mice were anesthetized by intraperitoneal injection of pentobarbital (120 mg/kg) and killed by cervical dislocation. The brains were removed and placed in artificial CSF (aCSF) (124 mM NaCl, 5 mM KCl, 1.3 mM MgCl₂ , 0.1 mM CaCl₂, 26 mM NaHCO₃, and 10 mM D-glucose, pH 7.3) aerated with 95% O2/5% CO₂ at room temperature. SVZ neural tissue - excluding subependyma - was isolated after coronal sectioning and cut into 1 mm³ pieces. Pieces were transferred into 30 ml of aCSF containing 1.3 mg/ml trypsin, 0.67 mg/ml hyaluronidase, and 0.2 mg/ml kynurenic acid (all from Sigma) and incubated, under continuous oxygenation and stirring, for 90 min at 32-34°C. Tissue sections were then rinsed in aCSF for 10 min, transferred to DMEM/F12 (Life Technologies) medium containing 0.7 mg/ml ovomucoid (Sigma), and carefully triturated with a fire-polished Pasteur pipette. Cells were collected by centrifugation and resuspended in GF-free, chemically defined DMEM/F12 medium containing 2 mM L-glutamine, 0.6% glucose, 9.6 mg/ml putrescine, 6.3 ng/ml progesterone, 5.2 ng/ml sodium selenite, 0.025 mg/ml insulin, 0.1 mg/ml transferrin, and 2 µg/ml heparin (Sigma). Cells were then cultured in NS-A medium (Euroclone) containing 20 ng/ml of epidermal growth factor (EGF) and 10 ng/ml fibroblast growth factor (FGF)-II (*growth medium*) (both from Peprotech). The number of primary spheres was counted after 7-12 days in vitro (DIV). For cell amplification, 8000 cells/cm² were plated at each sub-culturing passage in untreated tissue culture flasks. After 3-4 days (time estimated to obtain the doubling of cell number), neurospheres were harvested, mechanically dissociated, counted and re-plated under the same culture conditions. aNPCs at passage number ≤ 15 were used in all *in vivo* and *in vitro* experiments.

### R-EAE induction

SJL mice (Charles-River) were immunized with 200µg PLP139-151 (Espikem) in complete Freund's adjuvant (CFA), as described (McRae, B. L. et al. Induction of active and adoptive relapsing experimental autoimmune encephalomyelitis (EAE) using an encephalitogenic epitope of proteolipid protein. J Neuroimmunol 38, 229-40 (1992)). Body weight and clinical score (0= healthy; 1= limp tail; 2= ataxia and/or paresis of hind limbs; 3= paralysis of hind limbs and/or paresis of forelimbs; 4= tetra paralysis; 5= moribund or death) were recorded daily. Clinical relapses were defined as the occurrence of 0.5 increase of the clinical score persisting for a minimum of three consecutive days.

### aNPC transplantation

aNPCs were labelled *in vitro* using a third-generation lentiviral vector pRRLsin.PPT-hCMV engineered with the E. Coli-derived β-galactosidase (*LacZ*) gene containing a nuclear localization signal (*nls*) (Pluchino, S. et al. Injection of adult neurospheres induces recovery in a chronic model of multiple sclerosis. Nature 422, 688-94 (2003)). Single cell dissociated aNPCs (from 1 to 2 x10⁶ cells in 150µl PBS) were injected intravenously (i.v.) trough the tail vein. Sham-treated age-, sex- and strain-matched mice injected i.v. with PBS alone were used as controls. To assess in situ proliferation of i.v injected aNPCs, 106 dpi R-EAE mice were i.p.-treated with bromodeoxyuridine (BrdU, Roche, 50mg/kg) for three consecutive days and sacrificed soon thereafter, as previously described (McRae, B. L. et al. Induction of active and adoptive relapsing experimental autoimmune encephalomyelitis (EAE) using an encephalitogenic epitope of proteolipid protein. J Neuroimmunol 38, 229-40 (1992)). All procedures involving animals were performed according to the guidelines of the Animal Ethical Committee of our Institute (IACUC).

### Neuropathology

Paraffin embedded tissue section (5 µm) were stained with haematoxilin and eosin, Luxol fast blue and Bielshowsky to detect inflammatory infiltrates, demyelination and axonal loss, respectively (Lindvall, O., Kokaia, Z. & Martinez-Serrano, A. Stem cell therapy for human neurodegenerative disorders-how to make it work. Nat Med 10 Suppl, S42-50 (2004)). To detect in vivo i.v.-injected aNPCs two different protocols were used. (i) Fresh agarose-embedded CNS tissue sections (50-80 µm) were cut and incubated overnight at 37°C in 5-bromo-4-chloro-3- indolyl-β-D-galactoside (X-gal) solution to detect nuclear β-gal activity. β-gal⁺ agarose-embedded sections were then re-cut (5-7 µm) and processed for immunohistochemistry. (ii) β-gal⁺ cells were also detected on 5 µm frozen section by immunofluorescence using a mouse anti-β-galactosidase antibody (Promega). Nuclei were stained with 4'-6'-Diamidino-2-phenylindole (DAPI) (Roche). A total of 1204 β-gal⁺ cells were counted. Using both protocols, 40 brain and 70 spinal cord sections per mouse (taken at 100 µm intervals) were analysed; 5 mice per group were evaluated. Quantification of CNS damage was performed using IM-50 image analyser software (Leica) on a total of 315 spinal cord sections (21 sections per mouse, 5 mice per group). Confocal (Bio Rad, MRC 1024) and light (Olympus, BX51) microscopy was performed to analyse tissue stainings. Tissue sections processed for confocal imaging were analyzed at 0.5 µm intervals.

### List of antibodies

For immunofluorescence the following antibodies were used: rabbit anti-Ash 1 (Mash 1) (1:250, Chemicon International), rat anti-CDllb (MAC-1) (1:400, Abeam), rabbit anti-Dlx-2 (CeMines, Evergreen, CO, USA), goat anti-Jagged (1:100, Santa Cruz Biotechnology), rat anti-mouse CD45 (1:100, BD Biosciences), rat anti-mouse Embryonic NCAM (CD56) (1:100, BD Biosciences), rabbit anti-Laminin (1:500, Sigma), rat anti-mouse CD31 (1:100, BD Biosciences), rat anti-BrdU (1:40, Abeam), rabbit anti-neuronal class III β-Tubulin (1:1000, Covance), mouse anti- Nestin (1:500, Chemicon International), mouse anti-neuronal nuclei (NeuN) (1:1000, Chemicon International), rabbit anti-NG2 (1:100, Chemicon International), rabbit anti-GFAP (1:500, Dako), rabbit anti-PDGFr-a (1:1000, Santa Cruz Biotechnology), goat anti-BMP-4 (1:100, Santa Cruz Biotechnology), goat anti-Noggin (1:100, Santa Cruz Biotechnology), rabbit anti-Ki67 (1:1000, Novo Castra), rat anti-VCAM-1 (6 µg/ml, ATCC), rabbit anti-mouse active caspase-3 (1:250, BD Biosciences), and, rat anti-human CD3 (1:200, Serotec). Appropriate anti-rat, -mouse, -goat, -human and - rabbit fluorophore- (Alexa-fluor 488, 546; Molecular Probes) or biotin- (Amersham) conjugated secondary antibodies were used. For immunocytochemistry the following antibodies were used: mouse anti-mouse nestin (1:200, Chemicon), goat anti-mouse CCR2 (1:50), goat anti-mouse CCR5 (1:50), rabbit anti-mouse CXCR3 (1:100), rabbit anti-mouse CXCR4 (1:100, all from Santa Cruz Biotechnology), rat anti-mouse VLA-4 (20 µg/ml, PS/2, ATCC), rabbit anti-mouse FasL/CD95-ligand (1:10) and rabbit anti-mouse Tweak (1:10, both from Santa Cruz). Appropriate anti-mouse, -goat or - rabbit fluorophore-conjugated (Alexa-fluor 488, 546, 350, Molecular Probes) secondary antibodies were used.

### Intravital microscopy

Mabs anti-VCAM-1 (MK 2.7 and PS/2), anti-ICAM-1 (Y.N.1.7), and anti-MAdCAM-1 (MECA 367) and isotype-matched control abs were labelled using Alexa Fluor 488 labeling kit (Molecular Probes). The in vivo expression of VCAM-1 on brain and pectoral muscle microvessels was studied in MOG35-55-immunized C57BL/6 female mice - at 15, 24 and 35 dpi - and in LPS (Sigma)-treated mice (12 mg/kg i.p.) - 6 hours after injection.

### Immunocytochemistry

Single-cell dissociated or sphere-aggregated aNPCs were plated at 3 x10⁴ cells/cm² onto matrigel-coated glass chamber slides in *growth medium* and incubated 1 hour at 37°C. Fluorescent samples were analyzed with BioRad, MRC 1024 confocal image microscope. Image Pro plus software was used for VLA-4 distribution analysis.

### aNPC in vivo distribution study

aNPCs (2x10⁶ cells/mouse) were incubated 4 hours at 37°C, in *growth medium,* with 100µCi/ml 2,3-[³H]glycerol (MP Biomedicals), and then injected i.v. into both C57B1/6 mice - immunized with 200µg of myelin oligodendrocyte glycoprotein (MOG)35-55 (Espikem) in CFA (plus 500ng pertussis toxin)2- and SJL mice - immunized with PLP139-151 (McRae, B. L. et al. Induction of active and adoptive relapsing experimental autoimmune encephalomyelitis (EAE) using an encephalitogenic epitope of proteolipid protein. J Neuroimmunol 38, 229-40 (1992)). Mice were injected soon after disease onset and sacrificed 24 hours after transplantation. After perfusion, brain, spinal cord, kidney, spleen and liver were collected, weighted, sonicated and tissue radioactive content measured in a β-counter (LS1801; Beckman Coulter), as previously described (Constantin, G., Laudanna, C. & Butcher, E. C. Novel method for following lymphocyte traffic in mice using [3H]glycerol labeling. J Immunol Methods 203, 35-44 (1997)). Data are expressed as mean percentage of accumulated cells/gr. tissue (±SE) of at least 6 mice per group from a series of four independent experiments.

### Static adhesion assay

aNPCs were added at 70x10³/25µl/well on 18-well glass slides coated overnight at 4°C with (1µg/ml) purified mouse VCAM-1 (RAND D), as previously described (Constantin, G. et al. Chemokines trigger immediate beta2 integrin affinity and mobility changes: differential regulation and roles in lymphocyte arrest under flow. Immunity 13, 759-69 (2000)). Spleen-derived CD4+ T cells stimulated for 10 minutes with mitogens (phorbol 12-myristate 13-acetate [PMA]) (100ng/ml) were used as positive control. Activating pro-inflammatory chemokines (1µM) (i.e., CCL2/MCP-1, CXCL9/MIG, CXCL10/IP-10, CXCL11/I-TAC, CXCL12/SDF1α) were immediately added at 25x10³/25µl/well for 3 min at 37°C. Site density per square micrometer of immobilized VCAM-1 was calculated and data expressed as mean numbers (±SE) of adhered cells. Data are expressed as mean numbers of adherent cells (± SE) from a series of four independent experiments.

### Chemotaxis assay

The chemotactic response of aNPCs to different chemokines was evaluated using a modified 48-well microchemotaxis Boyden chamber system (Neuro Probe), as previously described (Lazarini, F. et al. Differential signalling of the chemokine receptor CXCR4 by stromal cell-derived factor 1 and the HIV glycoprotein in rat neurons and astrocytes. Eur J Neurosci 12, 117-25 (2000)).

### Apoptosis experiments

Spleen-derived CD3+ cells from naive SJL mice or encephalitogenic CD4⁺ PLP139-151-specific T cell lines were co-cultured *in vitro* with titrated concentrations of aNPCs for 18 hours at 37°C, 7% CO₂. T cells were then harvested and FACS analysis was performed to enumerate apoptotic and necrotic CD3⁺ cells using the appropriate antibodies. In vitro pre-treatment (30 min. at r.t.) of aNPCs with antibodies blocking death receptor ligands was performed using hrTRAIL-R2: Fc (10µg/ml), mrFn14:Fc (10µg/ml), or Fas:Fc (20µg/ml) (all from Alexis). IPNγ and iNOS were also blocked before performing apoptosis experiments using either a rabbit anti-mouse IFNγ blocking antibody (10µg/ml 30 min at r.t., Pharmingen) or by adding the 5-methylisothiourea sulfate (SMT) iNOS inhibitor (500 µM, Santa Cruz) to the co-culture wells, respectively. Data are expressed as mean percentage of positive cells over basal (±SE) of at least three mice per group from a minimum of three independent experiments for each protocol.

### Generation of PLP139-151-specific mouse Th1 and Th2 cell lines

PLP139-151- specific T cell lines were obtained from draining lymph nodes of PLP139-151- immunized SJL mice at 10 dpi, as described (Parras, C. M. et al. Mash1 specifies neurons and oligodendrocytes in the postnatal brain. Embo J 23, 4495-505 (2004)). Briefly, 5x10⁶ cells/ml were cultured at 37°C and 7% CO² in RPMI (Gibco) supplemented with 50 µM 2-β-mercaptoethanol (Gibco), 2mM L-glutamine (BioWhittaker), 1 mM Sodium Piruvate (BioWhittaker), 1 mM penicillin (Gibco), 100 µg/ml streptomycin (Gibco), and 10% FCS (Sigma), in presence of 30µg/ml PLP139-151 (Espikem). After 14 days of in vitro expansion, T cells were re-stimulated for three days with 30 µg/ml PLP139-151 - in the presence of antigen presenting cells (APC) at a 1:5 T cells/APC ratio - and, then, characterized for either Th1 (IFNγ⁺/TNFα⁺/IL-4⁻) or Th2 (IFNγ⁻/TNFα⁻/IL-4⁺) cytokine production using intracellular staining followed by FACS analysis.

### Lympho/monocyte (LNC)/NPC co-cultures

For mouse in vitro co-culture systems, lympho/monocytes (LNC) for proliferation assays were obtained from the draining lymph nodes of PLP139-151-immunized SJL mice, at 10 days after immunization. LNC suspensions were prepared using 70µm cell strainers (Becton Dickinson, Franklin Lakes, NJ, USA). Cells were suspended in complete RPMI medium containing 2 mM glutamine (Sigma-Aldrich, St. Louis, MO, USA), 100 IU/mL penicillin, 100 µg/mL streptomycin (Biowhittaker, Cambrex, Belgium), 10% fetal bovine serum (FBS, Biowhittaker, Cambrex, Belgium), and 50µM β-Mercaptoethanol (Sigma-Aldrich, St. Louis, MO, USA). All cultures were carried out in triplicate. The proliferation of LNCs was assayed in vitro by 3H-thymidine incorporation. The assay was carried out by seeding 7x105 cells/well in 200 µl RPMI medium supplemented with 10% fetal calf serum, 1 µM L-glutamine and antibiotics. Basal 3H-thymidine incorporation was examined, as well as in response to 1-30 µg/ml of PLP139-155 peptide (Espikem, Florence, Italy). To examine the effects of aNPCs at time of antigen presentation, single-cell dissociated mouse NPCs were transferred to the 96 well plates (1:2 NPC/LNC ratio) and then and co-cultured with LNCs. Supernatants were collected from 3-day cultures and T-cell proliferation was studied in RPMI medium by standard 3H-thymidine incorporation assay as early as after 72 hours of incubation. Cells were harvested on fiberglass filters using a multiharvester (Dynatech Laboratories, Alexandria, VA, USA) and radioactivity was measured by standard scintillation technique.

### Adoptive transfer of EAE

For adoptive transfer, following the in vitro co-culturing period, WT SJL 6-8 week-old females received 1×10⁶ LNC (which had previously been either co-cultured or not with syngeneic aNPCs) injected intravenously. Clinical EAE was graded daily from 0 to 5, as described.

### RT-PCR

Real-time quantitative PCR was performed using pre-developed Taqman™ Assay Reagents on an ABI Prism™ 7700 Sequence Detection System (Applied Biosystems) according to manufacturers protocol. Cell samples for relative quantification were collected. Two million cell samples were lysed in lysis buffer (Qiagen) and stored at - 80°C until the RNA was extracted following manufacture's instructions. Residual genomic DNA was removed by incubating with Dnase I, RNase-free (Qiagen) and eluted from the RNeasy mini columns with RNase-free water. The amount of total RNA was quantified using a NanoDrop ND-100 (Nano Drop Technologies) and the cDNA was synthesized from 5 µg of total RNA using the Ready-to-Go kit (Amersham) and a mixture of random examers (pd(N)6) used as primer. After purification using QIAquick PCR Purification kit (Qiagen), the c DNA samples were diluted 1:6 prior use in QPCR. Twenty-five ng of cDNA were used for Real-time PCR using pre-developed Taqman Assay Reagents (Applied Biosystems). Real-time quantitative PCR was performed with an ABI Prism™ 7700 Sequence Detection System (Applied Biosystems) according to manufacturers protocol. We used the housekeeping gene, GAPDH, as normalizer and ConA (5µg/ml for 72 hours) -activated splenocytes (from strain-, sex- and age-matched mice) as calibrator. Data were generated using aNPC samples obtained from three different non-immortalized aNPC lines at ≤ 10 passages of in vitro amplification. Results (mean of three independent experiments) are expressed as arbitrary units (AU) and represent fold induction (mean ± SD) of mRNA levels detected in aNPCs over ConA-activated splenocytes, which were used as positive internal calibrator.

### Cytokine and chemokine assay

We measured Th1/Th2 cytokines (IL-2, IL-4, IL-10, IFNγ, TNFα). These molecules were determined with multiple immunochemiluminescence ELISAs (SearchLightTM, Pierce Biotechnology Inc., Rockford, IL, USA), in accordance with the manufacturer's instructions. Each well of the ELISA microplate was pre-spotted with nine Th1/Th2 cytokine-specific (mouse Th1/Th2 Array), capture antibodies. Co-culture supernatants were ten-fold diluted with Sample Diluent for the mouse Th1/Th2 array. Fifty µL of standards and supernatants were added to the wells. After 1 hour of incubation and triple thorough washing (with Wash Buffer), 50 µL of Biotynilated Antibody Reagent was added. After 30 minutes of incubation and subsequent washing, 50 µL of Streptavidin-HRP reagent was added to each well. After 30 minutes, 50 µL of SuperSignal® Substrate (1:1 mixture of Stable Peroxide and SuperSignal® Luminol Enhancer) was added. Luminescence was read with a cooled CCD camera (FluorChemTM 8000, Alpha Innotech). Array VisionTM software was used for the analysis of SearchLightTM images. Detection limits were as follows: IL-2, 0.2 pg/mL; IL-4, 0.4 pg/mL; IL-10, 0.2 pg/mL; IFNγ, 0.2 pg/mL; TNFα, 1.6 pg/mL. All samples were analysed in duplicate. Intra- and inter-assay imprecision was below 15% (manufacturer's instructions).

### FACS analysis

Cell staining with different combination of labelled antibodies - recognizing CD3, CD4, B7.1, B7.2, CTLA-4, FasL and Tweak (Santa Cruz), IL-4, IPNγ and TNFα (Caltag Laboratories) - was used in combination with Annexin-V, TOPRO-3, PI (all from Pharmingen) staining.

### Flow cytometry and cell preparation

Fluorescein isothiocyanate (FITC)-, phycoerythrin (PE)-, or allophycocyanin (APC)-phycoerythrin Cyanine5.5 (Cy) conjugated antibody against mouse CD4, CD27, CD44, CD62L, PSGL-1, human CD4, CD27, CD44, CD62L, PSGL-1, human CD56/NCAM, leucocyte function associated (LFA)-1, CD44, intercellular cell adhesion molecule (ICAM)-1 and -2, α1, α2, α6, αv, β1, β2, β7 integrins and human CCR2, CCR3, CCR4, CCR5, CCR6, CCR7, CCR9, CXCR1, CXCR3, CXCR4 have been used. Both primary as well as appropriate isotype control antibodies were purchased from BD PharMingen (San Diego, CA, USA). Fluorescence-activated cell sorting (FACS) analysis was carried out on a FACSCalibur and FACSCanto instruments (both from BD Bioscience, San Jose, CA). For intracellular cytokine staining, cells from co-culture experiments were incubated at 37°C and 5% CO2 with Brefeldin A (1 µg/ml, Sigma, St. Louis, MO, USA), Ionomicin (1 µg/ml, Sigma, St. Louis, MO, USA), PMA (1 µg/ml, Sigma, St. Louis, MO, USA), for the 5 hrs. Cells were then washed, stained with anti-CD4, fixed in 3.7% formaldehyde, permeabilized with 0.5% saponin in PBS, and incubated with anti-IFNy, anti IL-4, IL-2, or anti IL10. Data was collected using a FACSCanto flow cytometer (BD Biosciences) and analyzed using FlowJo, version 6.1.1 (Tree Star).

### Example 2 - Therapeutic potential of subventricular zone (SVZ)-derived syngenic adult NPCs

We assessed the therapeutic potential of subventricular zone (SVZ)-derived syngenic aNPCs in a mouse model of chronic-recurrent autoimmune CNS inflammation - namely relapsing-remitting experimental autoimmune encephalomyelitis (R-EAE) - encompassing, as a long-term consequence of the repeated inflammatory episodes, neurodegenerative features such as demyelination and axonal loss (McRae, B. L. et al. J Neuroimmunol 38, 229-40 (1992)). SJL mice with R-EAE were intravenously (i.v.)-injected with β- galactosidase (β-gal)-labelled aNPCs (1x10⁶ cells/mouse) either at the first disease episode (13.1±0.3 days post immunization [dpi]) or at the occurrence of first clinical relapse (30.9±1.1 dpi). Mice were followed up to three months post-transplantation (p.t.). Clinical amelioration was observed using both treatment protocols (Table 1 and Figure 1). Mice transplanted at disease onset started to recover between 30 and 60 dpi, a period in which they developed significantly 2-fold less clinical relapses (p≤ 0.05), when compared to sham-treated mice. A similarly low relapse rate was maintained up to the end of the follow-up (106 dpi) (p≤ 0.05), when compared to sham-treated mice. Mice transplanted at the onset of the first relapse started to recover later, but showed a dramatic 3-fold reduction of the relapse rate between 60 and 90 dpi (p≤ 0.005, when compared to sham-treated mice). At the end of the follow up period, both groups of mice showed a significantly lower R-EAE cumulative score and a significant reduction (from 58 to 80%) of the extent of demyelination and axonal loss, when compared to sham-treated mice.

Irrespective from whether aNPCs had been injected at either R-EAE onset or at the occurrence of 1st clinical relapse, numerous β-gal⁺ cells persisted within the CNS (Figures 2 and 3). No β-gal activity was found in the CNS of shamtreated mice (including inflamed vessel walls) (Figure 3). At 106 dpi, we found 2.1 (±0.2) β-gal⁺ cells/mm² in mice transplanted at disease onset and 4.1 (±0.5) β- gal⁺ cells/mm² in mice transplanted at first relapse. The great majority of β-gal⁺ cells were located around inflamed CNS deep blood vessels, in close contact with blood-borne CNS-infiltrating CD45⁺ inflammatory immune cells (Figure 2 a and b), and maintained a round-shaped morphology typical of immature neural precursors (Figures 2 and 3). Some of β-gal⁺ cells were nestin⁺ (0.28 ±0.1 cell/mm²) (Figure 2c and d), NeuN⁺ (0.21 ±0.1 cell/mm²) (Figure 2e and f) or distal-less-related (Dlx)-2⁺ (0.12 ±0.1 cell/mm²) (Figure 2h and i), while few of them were immunoreactive for the pro-neural transcription factor mammalian achaete-scute homolog (Mash)-1 (Parras, C. M. et al. Embo J 23, 4495-505 (2004)). (Figure 2 g), or for the polysialylated neural cell adhesion molecule (PSA-NCAM) (Fukuda, S. et al. J Neurosci 23, 9357-66 (2003)). (Figure 2 j-m). None of the β-gal⁺ cells showed immunoreactivity for NG2, glial fibrillary acidic protein (GFAP) (Figure 4), β-tubulin III, or platelet-derived growth factor (PDGF) receptor-α. Occasionally, transplanted aNPCs displaying a stream-like tubular pattern of chain migration - reminiscent of that described for migrating neuroblasts within stem cell niches of the adult brain - were found in close contact with the blood vessel wall (Figure 3). To better define dynamic of aNPCs persisting within the CNS, R-EAE mice were treated with BrdU at 106 dpi. A consistent fraction of transplanted β-gal⁺ cells were BrdU⁺: 7.3% [±1.5] in mice transplanted at disease onset, and 8.4% [±1.4] in mice transplanted at 1^{st} relapse (Figure 2 n). Occasionally, β-gal⁺/BrdU⁺cells expressed early differentiation markers (e.g. PSA-NCAM) (Figure 4). Although long-term BrdU labelling study would have been proving similarity of perivascular CNS areas from R-EAE mice with stem cell niches in adult brain and definitive neural stem/progenitor cell markers are still lacking, the description of a consistent subpopulation of proliferating i.v.-injected cells - still maintaining an (early) differentiated phenotype around blood vessels - strongly suggests that inflamed CNS perivascular areas may function during R-EAE as ideal, although 'atypical', niche-like areas where transplanted cells can survive for long-term (up to 3 months p.t.) as 'bona fide' aNPCs.

### Example 3 - Mechanism(s) favouring aNPC survival within inflamed perivascular CNS areas

We then analysed putative mechanism(s) favouring aNPC survival within inflamed perivascular CNS areas. Irrespective of aNPC transplantation, major stem cell regulators and growth factors - involved both in angiogenesis and neurogenesis (i.e., bone morphogenetic proteins [BMPs], Noggin, Notch-I/Jagged-1, vascular endothelial growth factors [VEGF]-α - were found, at protein level, within these areas up to 106 dpi (Figure 5). BMP-4 and Noggin (Figures 6 and 7) were secreted not only by β-gal⁻ /GFAP⁺ astrocytes (Figure 6 a and b) and β-gal⁻ /Laminin⁺ endothelial cells (Figure 6 c and d), but also by blood-borne CNS-infiltrating CD45⁺ inflammatory cells (Figure 6e). This latter result was confirmed, at mRNA level, on spleen-derived lymphocytes (Figure 6 f).

### Example 4 - Cellular and molecular basis by which i.v.-injected aNPCs selectively reached inflamed perivascular CNS areas from R-EAE mice

We then explored cellular and molecular basis by which i.v.-injected aNPCs selectively reached inflamed perivascular CNS areas from R-EAE mice. Irrespective of the mouse strain (C57B1/6 vs. SJL), we found that half (53±10%) of aNPCs express α4 integrin very late antigen (VLA)-4 as constitutively activated molecule organized in clusters (12±5% cells were negative, while 31±9% had a "disperse" distribution) (Figure 8 a and b), a finding similar to that described in immune cells (Constantin, G. et al. Immunity 13, 759-69 (2000)). aNPC spontaneous adhesion to purified vascular cell adhesion molecule (VCAM)-1 - the VLA-4 counterligand - was basally high and comparable to that obtained with mitogen-activated CD4⁺ cells (Figure 8 c). However, adhesion was not significantly increased after stimulation with chemokines such as CCL2/MCP-1, CXCL9/MIG, CXCL10/IP-10, CXCL11/ITAC, and CXCL12/SDF-1α (Figure 8 c). The spontaneous ability of aNPCs to adhere to VCAM-1 expressing cells was confirmed using intravital microscopy in a subacute C57B1/6 mouse model of brain inflammation in which up-regulation of VCAM-1 expression on microvessels was obtained by i.p. injection of lipopolysaccharide (LPS) (Constantin, G. et al. J Immunol 162, 1144-9 (1999)) (Figure 8 d). This finding was further supported by the specific binding of aNPCs to venules from the pectoral muscle ectopically over-expressing VCAM-1 (Figure 8 e-g). A monoclonal antibody against VCAM-1 blocked more than 60% of aNPCs stable adhesion (both in the brain and in the muscle) while isotype-matched, anti-ICAM-1 and anti-MAdCAM-1 antibodies have no significant effect.

To verify the relevance of this phenomenon in R-EAE, ³H-glycerol-labelled aNPCs (2x10⁶ cells/mouse) were i.v.-injected into proteolipid protein (PLP)139-151-immunized SJL mice at the time of the first disease episode. As soon as 24 hours after cell injection, aNPCs were found into various bodily organs, while 3.1% (±0.2) of the cells accumulated within the CNS (Figure 8 h). A significant reduction of cell recruitment within the CNS (39-54%; p≤ 0.005) was obtained after *in vitro* pretreatment of aNPCs with an anti-VLA-4-blocking antibody (Figure 8 h). This effect was EAE specific since VLA-4 blocking did not induce any drop of cell recruitment into the CNS (and other peripheral organs) of naive mice i.v.-injected with labelled aNPCs (Figure 8 h). Results were further confirmed in a chronic progressive model of EAE obtained in C57B1/6 mice (Figure 9).

*In vitro* studies showed that pro-inflammatory chemokines do not further activate basal avidity for counter-ligands of VLA-4 expressed on aNPCs. However, we cannot exclude that culturing protocols with mitogens might somehow influence VLA-4 basal avidity on aNPCs *in vitro.* Thus, G-protein coupled receptor (GPCR) maybe then necessary in sub-optimal conditions - such as those possibly occurring in vivo (Alt et al. Eur J Immunol 32, 2133-44 (2002); Karpus et al. J Leukoc Biol 62, 681-7 (1997))-to further increase aNPC transendothelial migration or to mediate environmental positioning. We found that aNPCs express a wide range of pro-inflammatory chemokine receptors, at both mRNA and protein levels. Most cells within neurospheres express CCR1, CCR2, CCR5, CXCR3 and CXCR4, but do not express CCR3 and CCR7 (Figure 8 i-m). These receptors were functionally active since aNPCs responded with significant dose-dependent chemotaxis to CCL5/Rantes and CXCL12/SDF-1α (Figure 10). The response was clearly GPCR-dependent as it was completely inhibited by aNPC pre-treatment with the Gi-protein blocker *pertussis toxin* (Figure 10). Thus, aNPCs may use GPCRs - along with CAMs - to further improve their migratory capacity toward CNS inflamed lesions, and explain the partial inhibition on aNPCs extravasation obtained in R-EAE mice using an anti-α4 integrin-blocking antibody.

### Example 5 - Apoptosis of T-cells

It is apparent from the examples above that inflammation is acting as the "*danger signal*" determining both selective recruitment and long-term survival of i.v.-injected aNPCs in the CNS of R-EAE mice. Apoptosis is considered as one of the major mechanisms to promote recovery from EAE by inducing programmed cell death of CNS-infiltrating encephalitogenic T cells (Furlan, R. et al. J Immunol 167, 1821-9 (2001); Weishaupt, A. et al. J Immunol 165, 7157-63 (2000)). As early as 2 weeks p.t. (30 dpi), R-EAE mice transplanted at onset showed a significant reduction of the number of CNS inflammatory infiltrates (p≤ 0.01) and a three-fold increase of the number of CNS-infiltrating CD3⁺/TUNEL⁺ cells (p≤ 0.005, when compared to sham-treated mice) (Figures 11a and 12). In both aNPC- and sham-treated mice, the majority (83.8±6.9% vs. 90.5±2.3%) of apoptotic cells were confined within CNS perivascular inflammatory infiltrates. Results - confirmed using active caspase-3 as a marker for apoptosis - also demonstrated that transplanted β-gal⁺ cells, within perivascular CNS inflamed areas, did not undergo apoptosis (Figure 11 b). At 50 dpi, ex vivo FACS analysis - performed on CNS infiltrating CD3⁺ cells - showed a significantly (p=0.01) higher percentage (27.3±10.4%) of Annexin V⁺/TOPRO-3⁺ "*late apoptotic*" cells in R-EAE mice transplanted at onset, when compared to sham-treated mice (5.2±0.6%) (Figure 11 c). In the same mice, CNS infiltrating CD3⁺ cells did not show any sign of immunological anergy as indicated by the non-significant difference in the percentage of IFNγ⁺ (17.6±2.26%, sham-treated vs. 12.2±3.53% aNPC-treated), IL-2⁺ (17.8±1.64%, shamtreated vs. 33.6±10.7%, aNPC-treated), and IFNγ+/IL-2⁺ (59.3 ±1.96% sham-treated vs. 41.0 ±15.7%, aNPC-treated) producing cells. The pro-apoptotic effect of aNPCs on T cells was then confirmed by *in vitro* analyses. Spleen-derived CD3⁺ cells from naïve syngenic SJL mice - activated through plastic-immobilized anti-CD3/CD28 antibody - underwent apoptosis when co-cultured with increasing numbers of single cell dissociated aNPCs; in a trans-well system avoiding cell-to-cell contact, the proapoptotic effect of aNPCs on CD3⁺ cells was still measurable although less intense (Figure 12). Furthermore, PLP139-151-specific CD4⁺ T cell lines displaying a pro-inflammatory Th1 (e.g., TNFα, IFNγ) cytokine profile - but not PLP139-151-specific CD4⁺ T cell lines showing an anti-inflammatory Th2 (e.g., IL-4) profile- underwent apoptosis when *in vitro* co-cultured with increasing numbers of single cell dissociated aNPCs (Figure 11 d). Fibroblasts, whole bone marrow cells (WBMCs), or aNPC-conditioned medium did not induce any measurable pro-apoptotic effect when co-cultured with PLP139-151-specific Th1 cells.

*In vitro* and *in vivo* data suggest that aNPC-mediated apoptosis might be induced via both death receptor (*extrinsic*)- and mitochondrial (*intrinsic*)-mediated pathway (see, for a review, Marsden, V. S. & Strasser, A. Annu Rev Immunol 21, 71-105 (2003)). Blockade of death receptor ligands (e.g., FasL, Apo3L, TRAIL) - but not of IFNγ and inducible nitrix oxide synthase (iNOS) - significantly (22.3-50.1%) decreased apoptosis of PLP139-151-specific Th1 cells (Figure 11 d) thus indicating the predominant involvement of the death receptor pathway. However, we still cannot exclude the involvement of the mitochondrial apoptotic pathway. In fact, aNPCs - *in vitro* conditioned with pro-inflammatory cytokines (e.g., tumor necrosis factor [TNFα, IFNγ, and interleukin [IL]-1β- greatly increased (both at protein and mRNA level) not only the membrane expression of death receptor ligands (e.g. FasL, Trail, Apo3L) (Figure 11 e-g) but also the production of soluble factors potentially involved in mitochondrialmediated apoptosis (e.g., iNOS, IFNγ, glial-derived neurotrophic factor [GDNF] and leukaemia inhibitory factor [LIF]) (Weishaupt, A. et al. J Immunol 165, 7157-63 (2000); Marsden, V. S. & Strasser, A. Annu Rev Immunol 21, 71-105 (2003); Schere-Levy, C. et al. Exp Cell Res 282, 35-47 (2003); Tarrant, T. K. et al. J Exp Med 189, 219-30 (1999)) (Figures 12 and 13). Thus, our results indicate that aNPC-mediated T cells apoptosis occur via multiple distinct pathways, being the death receptor-mediated predominant. Finally, we found - at 106 dpi - increased numbers of process-bearing CD11b⁺ activated microglial cells (Figure 5) within perivascular CNS areas from aNPC-treated R-EAE mice (79.7 [±10.8] cell/mm² in mice treated at onset, 58.6 [±5.6] cell/mm² in mice treated at 1st relapse, 3.3 [±0.8] cell/mm² in sham-treated mice, p<0,001). Indeed, microglial cells were found capable of producing pro-apoptotic substances, upon *in vitro* activation with pro-inflammatory cytokines (Figure 12). Thus, we have to consider that CNS-resident glial cells might also contribute to promote T cell apoptosis, as previously suggested (Pender, M. P. & Rist, M. J. Glia 36, 137-44 (2001)).

Here we demonstrate that - upon systemic injection - undifferentiated aNSCs (including aNPCs) promote brain repair by exerting previously unidentified immune-like functions. Our data clearly indicate that the CNS microenvironment dictates the fate of systemically transplanted aNSCs and - as a consequence - their therapeutic efficacy. When neurodegeneration prevails, transplanted cells acquire a mature functional phenotype thus replacing damaged neural cells (pluchino et al. (2003) Nature 422:688-694). When neuroinflammation predominates - as we show here - transplanted aNSCs survive to recurrent inflammatory episodes by retaining both an undifferentiated phenotype and proliferating capacities. In this latter circumstance, inflammation represents the key "*danger signal*" orchestrating recruitment as well as long-term persistence of aNSCs within areas of CNS damage. Recruitment is possible because of the innate capacity of "circulating" aNSCs to recapitulate selected molecular pathways (e.g. VLA-4, GPCRs) used by bloodborne/ derived lymphocytes to patrol the CNS, while long-lasting CNS persistence is due to a continuous cross-talk occurring, within perivascular niche-like areas, between transplanted aNSCs and inflammatory CNS-infiltrating T cells - as well as CNS resident cells forming the inflammatory infiltrate - producing glio- and neuro-genic regulators (i.e. Notch-I and III, Noggin, VEGF-α). In these areas, aNSCs survive undifferentiated and exert their neuroprotective effect by inducing in situ programmed cell death of blood-borne CNS-infiltrating pro-inflammatory Th1 - but not anti-inflammatory Th2 - cells (Vandenbark, A. A. et al. Int Immunol 12, 57-66 (2000); Zhang, X. et al. J Exp Med 185, 1837-49 (1997)).

### Example 6 - Study of the molecular bases of NPC-dependent immunomodulation.

We have found that transplantation of undifferentiated aNPCs promotes long-lasting neuroprotection in experimental multiple sclerosis by remyelination of injured CNS axons as well as by immunomodulatory functions. We have also found that after systemic transplantation, aNPCs enter brain and spinal cord, selectively reach inflamed CNS areas - the "atypical perivascular niches" - where major stem cell regulators are focally (re)expressed - and survive in vivo for up to 120 days after transplantation. Within these CNS sites of accumulation and persistence, i.v.-injected aNPCs significantly exert neuroprotection by inducing in situ programmed cell death of blood-borne CNS-infiltrating pro-inflammatory Th1, but not anti-inflammatory Th2 cells.

To study in more details the molecular bases of aNPC-driven immunomodulation in experimental MS, we transplanted syngenic aNPCs into mice with the relapsing-remitting form of experimental autoimmune encephalomyelitis (R-EAE). SJL female mice were immunized subcutaneously with proteolipid protein (PLP)139-151 and injected intravenously with aNPCs at the onset of the disease.

In parallel to injected aNPCs accumulating within the CNS (namely brain and spinal cord), considerable amounts of transplanted aNPCs accumulated and persisted over 100 days after transplantation within major secondary lymphoid organs (Figure 14). Interestingly, within lymph nodes, the very same stem cell regulators (e.g., FGF-II, Notch I, BMPs, etc.) previously described at site of "atypical CNS perivascular niches" appeared to be dynamically - namely, in response to clinically-evident inflammatory episodes - expressed at both protein and mRNA levels (Figure 15).

Thus, systemically-injected aNPCs may survive in vivo even for long periods of time owing to their capability of accumulating and persisting within both canonical central (e.g. the CNS) as well as non-canonical peripheral (e.g., lymph nodes) bodily site(s), where major stem cell surviving factors are ectopically (re)expressed in response to inflammation.

To further explore the immunomodulatory properties of aNPCs at time of antigen presentation - as it is the case occurring for those i.v.-injected aNPCs accumulated within lymph nodes of R-EAE mice - we have developed an in vitro system into which CD4⁺ cell from lymph nodes of PLP139-151-immunized SJL mice are co-cultured with syngeneic antigen presenting cells (APC) and aNPCs.

Within this co-culture system,
i. At first challenge with the nominal antigen, aNPCs modulate polarization and antigen-specific activation of co-cultured CD4+ cells by inhibiting antigen-specific proliferation in a dose-dependent fashion (Figure 16);
ii. As net effect of the above, multiple immunochemiluminescence ELISAs confirmed that PLP-reactive T cells co-cultured with aNPCs have acquired (and maintained over in vitro culturing) a clear Th2-like phenotype. As a matter of fact, PLP-reactive T cells co-cultured with aNPCs release putative anti-inflammatory cytokines, such as IL-4 and IL-10, at significantly higher levels than non co-cultured counterparts, while down regulating the production of putative Th1-like pro-inflammatory cytokines (e.g., IFN-γ) (Figure 16);
ii. As a final proof, PLP 139-151-reactive T cell lines co-cultured with syngeneic aNPCs resulted significantly less encephalitogenic than non co-cultured counterpart, when studied in a EAE adoptive transfer setting in vivo (Figure 17).

Altogether, these in vivo and in vitro data show that aNPCs have a common functional immune-like signature, which may allow them to exert critical immunomodulatory functions responsible for the induction of both central as well as peripheral tolerance in MS as well as other CNS inflammatory diseases.

## Claims

1. An adult neural stem cell (aNSC) for use in the treatment of inflammation associated with central nervous system disorders.

2. The aNSC for use according to claim 1 wherein the use is in inducing central and/or peripheral tolerance in the central nervous system, or in a central nervous system inflammatory and/or a neurodegenerative disorder.

3. The aNSC for use according to claim 1 wherein the use is in inducing apoptosis of central nervous system infiltrating pro-inflammatory T cells.

4. The aNSC for use according to claim 3 wherein the pro-inflammatory T cells are CD45⁺ inflammatory cells.

5. The aNSC for use according to any preceding claim wherein the aNSC is an adult neural precursor cell.

6. The aNSC for use according to any one of claims 1 to 4 wherein the aNSC is derived from adult brain or spinal cord.

7. The aNSC for use according to any preceding claim wherein the aNSC comprises a targeting moiety for a site of inflammation.

8. The aNSC for use according to claim 7 wherein the site of inflammation is a central nervous system inflamed lesion.

9. The aNSC for use according to claim 7 or 8 wherein the aNSC is genetically modified to express the target moiety.

10. The aNSC for use according to any one of claims 7 to 9 wherein the targeting moiety is an integrin, a cell adhesion molecule (CAM), or a functional chemokine receptor that allows for the selective targeting of an inflamed area.

11. The aNSC for use according to claim 10 wherein the integrin is α4 integrin very late antigen (VLA)-4.

12. The aNSC for use according to claim 10 wherein the CAM is CD44.

13. The aNSC for use according to claim 10 wherein the chemokine receptor is selected from the group comprising CCR2, CCR5, CXCR3 and CXCR4.

14. The aNSC for use according to any preceding claim wherein the aNSC expresses a pro-apoptotic molecule.

15. The aNSC for use according to claim 14 wherein the aNSC is genetically modified to express the pro-apoptotic molecule.

16. The aNSC for use according to claim 14 or 15 wherein the pro-apoptotic molecule is a major death receptor ligand.

17. The aNSC for use according to claim 16 wherein the major death receptor ligand is selected from FasL, Apo3L and TRAIL.

18. The aNSC for use according to any preceding claim wherein the aNSC is murine, human, porcine, feline or canine.

19. The aNSC for use according to any one of claims 1 to 18 wherein the treatment is given after onset of a central nervous system disorder.

20. The aNSC for use according to any preceding claim wherein the subject has a neurodegenerative disorder.

21. The aNSC for use according to any preceding claim wherein the central nervous system disorder is selected from the group consisting of dementia, multiple sclerosis, amyotrophic lateral sclerosis, Alzheimer's Disease, Huntington's Disease, Parkinson's Disease, brain tumour, spinal cord injury and ischemic stroke.

22. The aNSC for use according to claim 21 wherein the central nervous system disorder is selected from the group consisting of multiple sclerosis, brain tumour, spinal cord injury and ischemic stroke.

23. The aNSC for use according to any preceding claim wherein the aNSC is administered intravenously or intrathecally.

24. Use of an adult neural stem cell (aNSC) for the preparation of a medicament for the treatment of inflammation associated with central nervous system disorders.

25. The aNSC for use according to claim 1 or 2 wherein the aNSC is for administration in an inflammation-related time window in a patient suffering from a central nervous system disorder.

26. The aNSC for use according to claim 25 wherein the aNSC is as defined in any one of claims 5 to 23.

27. The aNSC for use according to claim 25 or 26 wherein the central nervous system disorder is an inflammatory and/or a neurodegenerative disorder.

28. The use according to claim 24 wherein the medicament is adapted for administration in an inflammation time-related window in a patient suffering from a central nervous system disorder.

## Patentansprüche

1. Adulte neurale Stammzelle (aNSC) zur Verwendung bei der Behandlung einer Entzündung im Zusammenhang mit Erkrankungen des zentralen Nervensystems.

2. aNSC zur Verwendung nach Anspruch 1, wobei die Verwendung beim Induzieren von zentraler und/oder peripherer Toleranz im zentralen Nervensystem oder bei einer entzündlichen und/oder einer neurodegenerativen Erkrankung erfolgt.

3. aNSC zur Verwendung nach Anspruch 1, wobei die Verwendung beim Induzieren von Apoptose das zentrale Nervensystem infiltrierender entzündungsfördernder T-Zellen erfolgt.

4. aNSC zur Verwendung nach Anspruch 3, wobei es sich bei den entzündungsfördernden T-Zellen um CD45⁺-Entzündungszellen handelt.

5. aNSC zur Verwendung nach einem vorhergehenden Anspruch, wobei es sich bei der aNSC um eine adulte neurale Vorläuferzelle handelt.

6. aNSC zur Verwendung nach einem der Ansprüche 1 bis 4, wobei die aNSC aus adultem Gehirn oder Rückenmark stammt.

7. aNSC zur Verwendung nach einem vorhergehenden Anspruch, wobei die aNSC eine Targeting-Gruppierung für eine Entzündungsstelle umfasst.

8. aNSC zur Verwendung nach Anspruch 7, wobei es sich bei der Entzündungsstelle um eine entzündete Läsion des zentralen Nervensystems handelt.

9. aNSC zur Verwendung nach Anspruch 7 oder 8, wobei die aNSC gentechnisch verändert ist, so dass sie die Zielgruppierung exprimiert.

10. aNSC zur Verwendung nach einem der Ansprüche 7 bis 9, wobei die es sich bei der Targeting-Gruppierung um ein Integrin, ein Zelladhäsionsmolekül (CAM) oder einen funktionellen Chemokin-Rezeptor handelt, was ein selektives Targeting eines entzündeten Bereichs ermöglicht.

11. aNSC zur Verwendung nach Anspruch 10, wobei es sich bei dem Integrin um α4-Integrin VLA(Very Late Antigen)-4 handelt.

12. aNSC zur Verwendung nach Anspruch 10, wobei es sich bei dem CAM um CD44 handelt.

13. aNSC zur Verwendung nach Anspruch 10, wobei der Chemokin-Rezeptor aus der Gruppe umfassend CCR2, CCR5, CXCR3 und CXCR4 ausgewählt ist.

14. aNSC zur Verwendung nach einem vorhergehenden Anspruch, wobei die aNSC ein Apoptose förderndes Molekül exprimiert.

15. aNSC zur Verwendung nach Anspruch 14, wobei die aNSC gentechnisch verändert ist, so dass sie das Apoptose fördernde Molekül exprimiert.

16. aNSC zur Verwendung nach Anspruch 14 oder 15, wobei es sich bei dem Apoptose fördernden Molekül um einen MDR(Major Death Receptor)-Liganden handelt.

17. aNSC zur Verwendung nach Anspruch 16, wobei der MDR-Ligand aus FasL, Apo3L und TRAIL ausgewählt ist.

18. aNSC zur Verwendung nach einem vorhergehenden Anspruch, wobei es sich um eine aNSC von Maus, Mensch, Schwein, Katze oder Hund handelt.

19. aNSC zur Verwendung nach einem der Ansprüche 1 bis 18, wobei die Behandlung nach Ausbruch einer Erkrankung des zentralen Nervensystems erfolgt.

20. aNSC zur Verwendung nach einem vorhergehenden Anspruch, wobei bei dem Individuum eine neurodegenerative Erkrankung vorliegt.

21. aNSC zur Verwendung nach einem vorhergehenden Anspruch, wobei die Erkrankung des zentralen Nervensystems aus der Gruppe bestehend aus Demenz, multipler Sklerose, amyotropher Lateralsklerose, Morbus Alzheimer, Morbus Huntington, Morbus Parkinson, Hirntumor, Rückenmarksverletzung und ischämischem Schlaganfall ausgewählt ist.

22. aNSC zur Verwendung nach Anspruch 21, wobei die Erkrankung des zentralen Nervensystems aus der Gruppe bestehend aus multipler Sklerose, Hirntumor, Rückenmarksverletzung und ischämischem Schlaganfall ausgewählt ist.

23. aNSC zur Verwendung nach einem vorhergehenden Anspruch, wobei die aNSC intravenös oder intrathekal verabreicht wird.

24. Verwendung einer adulten neuralen Stammzelle (aNSC) zur Herstellung eines Arzneimittels für die Behandlung einer Entzündung im Zusammenhang mit Erkrankungen des zentralen Nervensystems.

25. aNSC zur Verwendung nach Anspruch 1 oder 2, wobei die aNSC für eine Verabreichung innerhalb eines entzündungsbezogenen Zeitfensters bei einem an einer Erkrankung des zentralen Nervensystems leidenden Patienten vorgesehen ist.

26. aNSC zur Verwendung nach Anspruch 25, wobei die aNSC die in einem der Ansprüche 5 bis 23 angegebene Bedeutung aufweist.

27. aNSC zur Verwendung nach Anspruch 25 oder 26, wobei es sich bei der Erkrankung des zentralen Nervensystems um eine entzündliche und/oder eine neurodegenerative Erkrankung handelt.

28. Verwendung nach Anspruch 24, wobei das Arzneimittel für eine Verabreichung innerhalb eines entzündungszeitbezogenen Fensters bei einem an einer Erkrankung des zentralen Nervensystems leidenden Patienten zugeschnitten ist.

## Revendications

1. Cellule souche neurale adulte (CSNa) pour utilisation dans le traitement de l'inflammation associée à des troubles du système nerveux central.

2. CSNa pour utilisation selon la revendication 1, où l'utilisation consiste en l'induction d'une tolérance centrale et/ou périphérique dans le système nerveux central, ou dans un trouble inflammatoire et/ou neurodégénératif du système nerveux central.

3. CSNa pour utilisation selon la revendication 1, où l'utilisation consiste en l'induction de l'apoptose des cellules T pro-inflammatoires infiltrant le système nerveux central.

4. CSNa pour utilisation selon la revendication 3, où les cellules T pro-inflammatoires sont des cellules inflammatoires CD45⁺.

5. CSNa pour utilisation selon l'une quelconque des revendications précédentes, où la CSNa est une cellule précurseur neurale adulte.

6. CSNa pour utilisation selon l'une quelconque des revendications 1 à 4, où la CSNa est dérivée de cerveau ou de moelle épinière adulte.

7. CSNa pour utilisation selon l'une quelconque des revendications précédentes, où la CSNa comprend une entité ciblant un site d'inflammation.

8. CSNa pour utilisation selon la revendication 7, où le site d'inflammation est une lésion inflammatoire du système nerveux central.

9. CSNa pour utilisation selon la revendication 7 ou 8, où la CSNa est génétiquement modifiée pour exprimer l'entité cible.

10. CSNa pour utilisation selon l'une quelconque des revendications 7 à 9, où l'entité ciblante est une intégrine, une molécule d'adhésion cellulaire (CAM) ou un récepteur de chimiokine fonctionnel qui permet le ciblage sélectif d'une zone inflammatoire.

11. CSNa pour utilisation selon la revendication 10, où l'intégrine est l'antigène (VLA)-4 de l'intégrine α4.

12. CSNa pour utilisation selon la revendication 10, où la CAM est CD44.

13. CSNa pour utilisation selon la revendication 10, où le récepteur de chimiokine est choisi dans le groupe comprenant CCR2, CCR5, CXCR3 et CXCR4.

14. CSNa pour utilisation selon l'une quelconque des revendications précédentes, où la CSNa exprime une molécule pro-apoptotique.

15. CSNa pour utilisation selon la revendication 14, où la CSNa est génétiquement modifiée pour exprimer la molécule pro-apoptotique.

16. CSNa pour utilisation selon la revendication 14 ou 15, où la molécule pro-apoptotique est un ligand de récepteur de mort principal.

17. CSNa pour utilisation selon la revendication 16, où le ligand de récepteur de mort principal est choisi parmi FasL, Apo3L et TRAIL.

18. CSNa pour utilisation selon l'une quelconque des revendications précédentes, où la CSNa est murine, humaine, porcine, féline ou canine.

19. CSNa pour utilisation selon l'une quelconque des revendications 1 à 18, où le traitement est donné après le début d'un trouble du système nerveux central.

20. CSNa pour utilisation selon l'une quelconque des revendications précédentes, où le sujet souffre d'un trouble neurodégénératif.

21. CSNa pour utilisation selon l'une quelconque des revendications précédentes, où le trouble du système nerveux central est choisi dans le groupe constitué par démence, sclérose en plaques, sclérose latérale amyotrophique, maladie d'Alzheimer, maladie de Huntington, maladie de Parkinson, tumeur cérébrale, lésion de la moelle épinière et accident ischémique.

22. CSNa pour utilisation selon la revendication 21, où le trouble du système nerveux central est choisi dans le groupe constitué par sclérose en plaques, tumeur cérébrale, lésion de la moelle épinière et accident ischémique.

23. CSNa pour utilisation selon l'une quelconque des revendications précédentes, où le CSNa est administré par voie intraveineuse ou intrathécale.

24. Utilisation d'une cellule souche neurale adulte (CSNa) dans l'élaboration d'un médicament destiné au traitement d'une inflammation associée à des troubles du système nerveux central.

25. CSNa pour utilisation selon la revendication 1 ou 2, où la CSNa est destinée à l'administration dans un intervalle de temps lié à l'inflammation chez un patient souffrant d'un trouble du système nerveux central.

26. CSNa pour utilisation selon la revendication 25, où le CSNa est tel que défini dans l'une quelconque des revendications 5 à 23.

27. CSNa pour utilisation selon la revendication 25 ou 26, où le trouble du système nerveux central est un trouble inflammatoire et/ou neurodégénératif.

28. Utilisation selon la revendication 24, où le médicament est adapté à l'administration dans un intervalle de temps lié à l'inflammation chez un patient souffrant d'un trouble du système nerveux central.
